(19) Europäisches Patentamt
European Patent Office
Office européen des brevets

(11) Publication number: 0 251 536 A1

# (12) EUROPEAN PATENT APPLICATION

(21) Application number: 87305207.0

(22) Date of filing: 12.06.87

(51) Int. Cl.[4]: C07D 235/28 , C07D 491/04 , C07D 498/04 , A61K 31/415 , //C07C43/205,C07C79/35,C07C-95/08,C07C91/40

(30) Priority: 24.06.86 GB 8615416
24.06.86 GB 8615417
24.06.86 GB 8615418
24.06.86 GB 8615419
06.02.87 GB 8702683
06.02.87 GB 8702685
06.02.87 GB 8702686

(43) Date of publication of application: 07.01.88 Bulletin 88/01

(84) Designated Contracting States: AT BE CH DE ES FR GB GR IT LI LU NL SE

(71) Applicant: FISONS plc
Fison House Princes Street
Ipswich Suffolk IP1 1QH(GB)

(72) Inventor: Cox, David
3 Belvoir Drive
Loughborough Leicestershire, LE11 2SN(GB)
Inventor: Hall, David Edward
81 Brooke Street
Wymeswold Leicestershire, LE12 6TT(GB)
Inventor: Ingall, Anthony Howard
53 Forest Road
Loughborough Leicestershire LE11 3NW(GB)
Inventor: Suschitzky, John Louis
73 Kirkstone Drive
Loughborough Leicestershire(GB)

(74) Representative: Wright, Robert Gordon McRae
Fisons plc 12 Derby Road
Loughborough, Leicestershire LE11 0BB(GB)

## (54) Benzimidazoles, their production, formulation and use as gastric acid secretion inhibitors.

(57) There are described compounds of formula I,

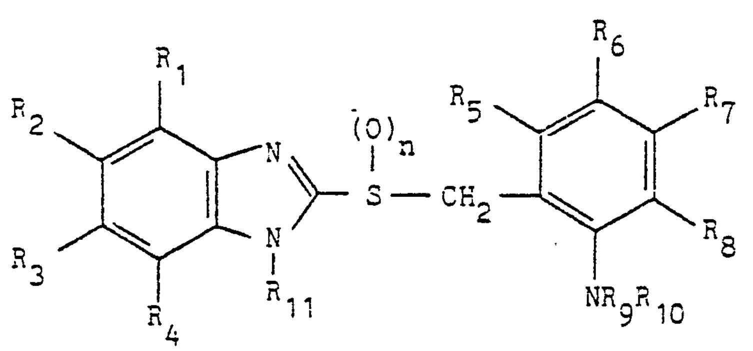

I

in which $R_1$ to $R_8$ have a variety of significances, including alkoxy, hydrogen, alkyl,
n is 0 or I,
$R_{11}$ is hydrogen, phenyl, alkenyl, or alkyl optionally substituted by phenyl,

EP 0 251 536 A1

$R_9$ and $R_{10}$ have a variety of significances, including cycloalkyl, alkyl, or may form a ring with the nitrogen atom to which they are attached,

and certain provisos.

Processes for making the compounds and pharmaceutical formulations containing them, eg for the treatment of conditions including excess gastric acidd secretion, are also described.

# BENZIMIDAZOLES, THEIR PRODUCTION, FORMULATION AND USE AS GASTRIC ACID SECRETION INHIBITORS

This invention relates to new compounds, methods for their preparation and pharmaceutical formulations comprising them.

A number of 2-(2-benzenamine sulphinylmethyl) benzimidazoles are known for use as pharmaceuticals, eg from British Patent Application Nos 2,161,160 and 2,163,747.

We have now found a novel group of benzimidazoles which have pharmacological activity.

According to the invention we provide compounds of formula I,

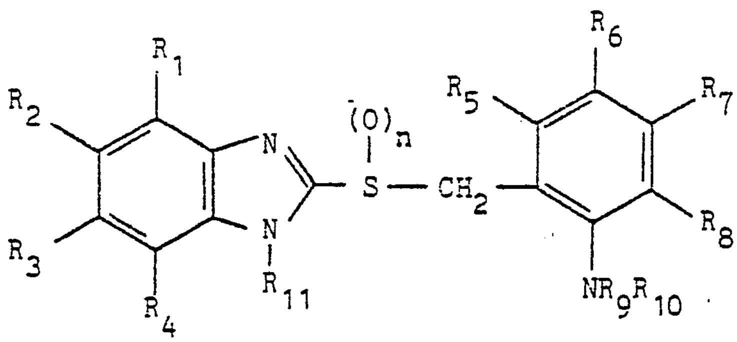

I

in which $R_1$, $R_2$, $R_3$, $R_4$, $R_5$, $R_6$, $R_7$ and $R_8$, which may be the same or different, are each hydrogen, halogen, alkyl C 1 to C6, $-(CH_2)_mOH$, $-NO_2$, $-NR_{13}R_{14}$, -COOH or an ester thereof, or alkoxy C 1 to C6 optionally substituted by a saturated heterocyclic ring,

or an adjacent pair of $R_1$, $R_2$, $R_3$ and $R_4$ may, in addition to the values given above, form an $-OCH_2CH_2O-$ or -OCONH-chain,

m is 0 or 1,

n is 0 or 1,

$R_{13}$ and $R_{14}$, which may be the same or different, are each hydrogen or alkyl C 1 to C6,

$R_{11}$ is hydrogen, phenyl, alkenyl C2 to C6, or alkyl C 1 to C6 optionally substituted by phenyl,

$R_9$ and $R_{10}$, which may be the same or different, are each cycloalkyl C3 to C7 or alkyl C 1 to C6, optionally substituted by phenyl, or

$R_9$ and $R_{10}$, together with the nitrogen atom to which they are attached, may form a saturated 6 to 8 inclusive membered ring which contains no heteroatoms other than the nitrogen atom to which $R_9$ and $R_{10}$ are attached,

provided that

a) when $R_{11}$ is hydrogen, then at least one of $R_1$, $R_2$, $R_3$, $R_4$ is other than hydrogen;

b) when $R_5$, $R_6$, $R_{11}$ are each hydrogen and $R_9$, $R_{10}$ are both methyl, then

i) when $R_1$, $R_3$, $R_4$, $R_7$, $R_8$ are each hydrogen, $R_2$ is not chloro, methoxycarbonyl, methyl, methoxy, $-NO_2$ or $-NH_2$;

ii) when $R_1$, $R_4$, $R_7$, $R_8$ are each hydrogen, $R_2$ and $R_3$ do not both represent methyl, chloro or methoxy;

iii) when $R_2$, $R_3$, $R_7$, $R_8$ are each hydrogen, $R_1$ and $R_4$ do not both represent methoxy;

iv) when $R_2$, $R_3$, $R_4$, $R_7$, $R_8$ are each hydrogen, $R_1$ is not methyl;

v) when $R_1$, $R_3$, $R_4$, $R_7$ are each hydrogen and $R_8$ is methyl, $R_2$ is not methoxy;

vi) when $R_1$, $R_3$, $R_4$, $R_8$ are each hygrogen and $R_7$ is methoxy, $R_2$ is not chloro;

c) when $R_1$ to $R_8$ each represent hydrogen, $R_9$, $R_{10}$, $R_{11}$ do not each represent methyl;

d) when $R_1$, $R_4$, $R_7$, $R_8$ and $R_{11}$ are each hydrogen, $R_9$ and $R_{10}$ are both ethyl, $R_2$ and $R_3$ are both methoxy and one of $R_5$ or $R_6$ is hydrogen the remainder of $R_5$ or $R_6$ is not hydrogen or methyl,

and pharmaceutically acceptable salts thereof.

According to the invention we also provide a process for the production of a compound of formula I, or a pharmaceutically acceptable salt thereof, which comprises

a) production of a compound of formula I in which n is 1 by selective oxidation of a compound of formula I

in which n is 0,

b) production of a compound of formula I in which $R_{11}$ is not hydrogen, by reaction of a corresponding compound of formula I in which $R_{11}$ is hydrogen with a compound $R_{11}Z$ in which $R_{11}$ is as defined above save that it can not be hydrogen, and Z is a good leaving group,

c) production of a compound of formula I carrying a $-NH_2$ group by selective reduction of a corresponding compound of formula I carrying a $-NO_2$ group, or

d) production of a compound of formula I in which n is 0, by reaction of a compound of formula II,

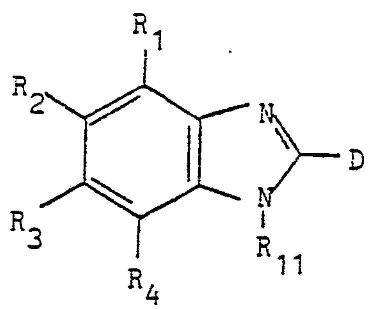

II

in which $R_1$, $R_2$, $R_3$, $R_4$ and $R_{11}$ are as defined above,

with a compound of formula III,

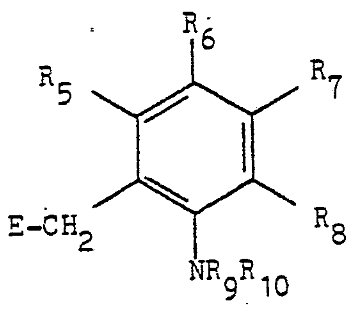

III

in which $R_5$, $R_6$, $R_7$, $R_8$, $R_9$ and $R_{10}$ are as defined above, and

one of D and E is -SH and the other is a good leaving group, eg halogen (chlorine or bromine),

and where desired or necessary converting the resulting compound of formula I to a pharmaceutically acceptable salt thereof, or vice versa.

The selective oxidation of process a) may be carried out in a solvent which is inert under the reaction conditions, eg ethyl acetate, dichloromethane, chloroform or a mixture thereof. The reaction is preferably carried out at less than room temperature, eg -20° to +20°C Suitable oxidizing agents for use in the reaction are peracids, eg m-chloroperbenzoic acid or t-butylhydroperoxide in the presence of a suitable catalyst, eg vanadylacetylacetonate, or periodates, eg sodium periodate in aqueous alcohol, eg methanol.

In process b) the good leaving group may be, for example, halogen (chlorine or iodine) and the reaction may be carried out in a solvent or solvent mixture which is inert under the reaction conditions, eg dimethylformamide, in the presence of a base, eg potassium carbonate, and at a temperature in the range 15-35°C, eg at 25°.

In process c) the selective reduction may, for example, be carried out chemically under basic conditions, eg using hydrazine and Raney nickel, but is preferably carried out using hydrogen and a catalyst, eg $PtO_2$, in ethanol as the reaction medium.

The reaction of process d) may be carried out in any suitable solvent, eg N,N-dimethyl formamide or N,N-dimethyl acetamide, at an optionally elevated temperature and may take place in the presence of a catalyst, eg Cu, or an acid acceptor, eg potassium carbonate.

Certain of the compounds of formulae II and III are novel and we provide these novel compounds as intermediates.

The compounds of formula II may be made from known compounds by conventional processes known per se, eg by reacting a compound of formula IV

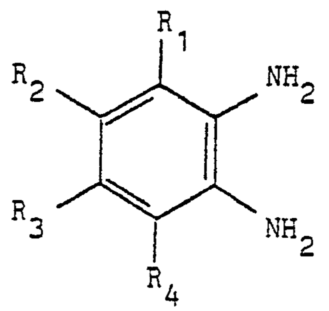

IV

in which $R_1$ to $R_4$ are as defined above,

with $CS_2$. The reaction is preferably carried out under nitrogen and at a temperature in the range 50-80°C.

The compounds of formula III may be made from known compounds by conventional processes known per se , eg by selective reduction of a compound of formula V

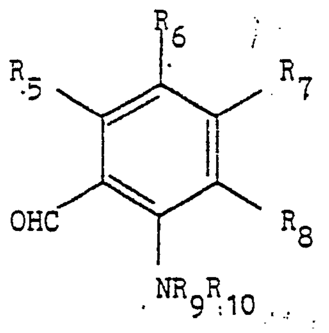

V

in which $R_5$ to $R_{10}$ are as defined above.

The selective reduction may be carried out in a solvent which is inert under the reaction conditions, eg ethanol or isopropanol. Suitable reducing agents for use in the reaction are $NaBH_4$, or $LiAlH_4$ in ether or tetrahydrofuran.

The compounds of formula I, and the intermediates therefor, may be isolated from their reaction mixtures using conventional techniques.

Pharmaceutically acceptable salts of the compounds of formula I include salts with suitable organic or inorganic acids, eg with a hydrohalic, sulphuric, alkanesulphonic, tartaric or citric acid. We also provide, when the compound of formula I carries a -COOH, or other acidic group, salts with suitable organic or inorganic bases, eg ammonium, alkali metal, alkaline earth metal, alkylamino, etc. salts. The benzimidazole nucleus itself is acidic and can form salts with appropriate bases as above.

We also provide the compounds of formula I, and pharmaceutically acceptable salts thereof, for use as pharmaceuticals, eg for use as cytoprotective agents, in the treatment or prophylaxis of inflammatory conditions, or in the prevention or inhibition of gastric acid secretion.

The compounds of formula I, and pharmaceutically acceptable salts thereof, are useful because they possess pharmacological activity in animals; in particular they are useful because they prevent or inhibit gastric acid secretion, eg in the test set out in Am. J. Physiol, l982, 243 (6), G505-5l0. The compounds of formula I are also useful as intermediates in the synthesis of other chemicals.

The new compounds are thus indicated for use in the prevention or inhibition of gastric acid secretion, and/or the treatment of conditions normally involving excess gastric acid secretion, eg peptic, duodenal, gastric, recurrent or stormal ulceration, dyspepsia, duodenitis, Zollinger-Ellison syndrome, reflux oesophagitis and the management of haemorrhage, eg from erosion of ulcers in the upper gastrointestinal tract, especially when a major blood vessel is not involved. The compounds may also be used to treat gastritis or dyspepsia associated with administration of non-steroidal anti-inflammatory drugs, in the prophylaxis of gastrointestinal haemorrhage from stress ulceration in seriously ill or burned patients, in the prophylaxis of recurrent haemorrhage in patients with bleeding peptic ulcers, before general anaesthesia in patients at risk of acid aspiration syndrome (Mendelson's syndrome) and to reduce the chance of haemorrhage in patients with leukaemia, graft versus host disease or with severe hepatic failure. The above conditions may be treated whether or not they are associated with excess gastric acid secretion.

The compounds may also be used to treat cholera, paratyphus, tourist diarrhoea, toxin-induced diarrhoea and local gastric catarrh.

The new compounds are also indicated for use as cytoprotective agents, especially for the gastrointestinal tract, and can be utilized for the treatment or prevention of a non-gastric-acid-induced, non-traumatically-induced, non-neoplastic gastrointestinal inflammatory disease for example, Crohn's disease, inflammatory bowel disease, infectious enteritis, colitis, ulcerative colitis, pseudomembranous colitis, diverticulitis, and allergenic and radiological inflammatory diseases.

The compounds are also indicated for use in the treatment of prophylaxis of inflammatory conditions in mammals, including man, especially those involving lysozymal enzymes. Conditions that may be specifically mentioned are: rheumatoid arthritis, gout, eczema, polyserositis and allergic alveolitis.

Patterns of therapeutic use which may be mentioned are:-

a) a high dose initially, for say 2-4 weeks, followed by lower-dose maintenance therapy after the condition has improved, eg the ulcer has healed,

b) as in a) above, but the maintenance therapy including another cytoprotective agent, eg a $PGE_2$ derivative,

c) combination therapy, using a low dose of the compound of the invention in association with a low, well-tolerated dose of another cytoprotectant and/or antacid,

d) intermittent dosing, eg every second day, may be appropriate as maintenance therapy.

For the above mentioned uses the dosage administered will, of course, vary with the compound employed, the mode of administration and the treatment desired. However, in general, satisfactory results are obtained when the compounds are administered at a dosage of from $10^{-6}$M to $10^{-4}$M in the test set out in Am.J.Physiol, 1982, 243 (6), G505-G510. For man the indicated total daily dosage is in the range of from about 1mg to 3,000mg, preferably 5 to 500mg, and more preferably from about 10mg to 200mg, which may be administered in divided doses from 1 to 6 times a day or in sustained release form. Thus unit dosage forms suitable for administration comprise from about 1.0mg to 600mg of the compound admixed with a solid or liquid pharmaceutically acceptable diluent, carrier or adjuvant.

The compounds of formula I, and pharmaceutically acceptable salts thereof, have one or more of the following advantages. They are more readily absorbed, have increased bioavailability, are more stable around neutral pH, are more specific in action, are more rapidly activated by acid, eg gastric acid, produce more advantageous results, eg in the 'Shay Rat Test' as described by H Shay et al in Gastroenterology, 5 43-61 (1945), are more stable to acid, eg gastric acid, or have other advantageous properties when compared to known compounds of similar structure.

When any of $R_1$ to $R_8$ is halogen we prefer it to be fluorine or chlorine.

When any of $R_1$ to $R_8$ represent an ester we prefer it to be with a C 1 to C6 alcohol, eg to be an ethyl or methyl ester.

Specific groups that $R_1$ to $R_8$ may represent include hydrogen, methoxycarbonyl, methyl, butyl, chloro, fluoro, methoxy, ethoxy, propyloxy, butyloxy, hydroxy, hydroxymethyl and $-NO_2$.

We prefer at least two of $R_1$ to $R_4$ to be alkoxy, in particular for at least two of $R_1$ to $R_4$ to be methoxy or ethoxy. We particularly prefer $R_2$ and $R_3$ to both be alkoxy, eg methoxy, or more particularly to both be ethoxy.

Specific adjacent pairs of $R_1$, $R_2$, $R_3$ and $R_4$ which form an $-OCH_2CH_2O-$or -OCONH-chain are $R_5$ -$R_6$ and $R_6$ -$R_7$.

When any of $R_1$ to $R_8$ represent alkoxy substituted by a saturated heterocyclic ring, we prefer that ring to be a morpholino ring, we particularly prefer that ring to be morpholino-N-oxide.

Specific groups $R_{11}$ include hydrogen, methyl, propyl, benzyl, phenyl, and prop-2-enyl.

We prefer $R_{11}$ to be hydrogen or alkyl C 1 to C6, eg methyl.

We prefer n to be 1.

Specific groups $R_9$ and $R_{10}$ include methyl, ethyl, propyl and cyclohexyl.

We prefer $R_9$ and $R_{10}$ to both be methyl, or for $R_9$ and $R_{10}$ together to comprise more than three carbon atoms. We particularly prefer at least one of $R_9$ and $R_{10}$ to be ethyl or more particularly for $R_9$ and $R_{10}$ to both be ethyl.

Preferably at least two of $R_1$ to $R_4$ are alkoxy C 1 to C6 and $R_9$ and $R_{10}$, which may be the same or different, are alkyl or together comprise more than 3 carbon atoms, more preferably at least two of $R_1$ to $R_4$ are selected from methoxy or ethoxy and $R_9$ and $R_{10}$, which may be the same or different, are methyl, ethyl or together comprise more than 3 carbon atoms and most preferably $R_2$ and $R_3$ are both alkoxy, eg both methoxy or both ethoxy, and $R_9$ and $R_{10}$ are both methyl, or together comprise more than 3 carbon atoms, eg are both ethyl.

When $R_9$ and $R_{10}$, together with the nitrogen atom to which they are attached, form a ring, we prefer that ring to be a piperidino ring.

Specific groups of compounds of formula I include

a)

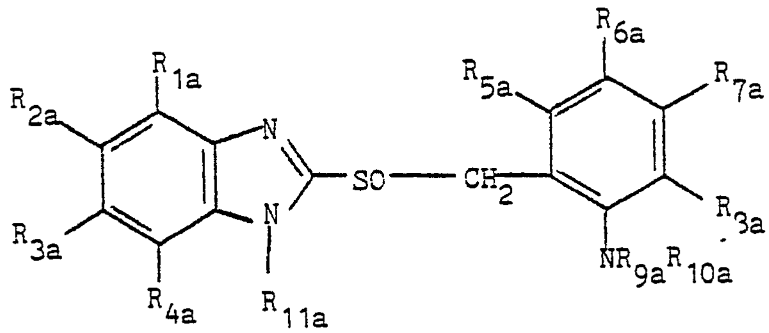

Ia

in which at least one of $R_{1a}$ , $R_{2a}$, $R_{3a}$ and $R_{4a}$ is alkoxy C I to C6, and the remainder, which may be the same or different are each hydrogen, halogen, alkyl C I to C6, or -COOH or an ester thereof,

$R_{11a}$ is hydrogen, alkenyl C2 to C6, or alkyl C I to C6 optionally substituted by phenyl,

$R_{5a}$, $R_{6a}$, $R_{7a}$ and $R_{8a}$ which may be the same or different, are each hydrogen, halogen, alkoxy C I to C6, alkyl C I to C6, $-NO_2$, or $-NR_{13}R_{14}$,

$R_{9a}$ and $R_{10a}$, which may be the same or different, are each cycloalkyl $C_3$ to $C_7$ or alkyl C I to C6 optionally substituted by phenyl,

$R_{13}$ and $R_{14}$ are as defined above,

provided that

a) when $R_{9a}$ and $R_{10a}$ are each both methyl and $R_{5a}$ , $R_{6a}$, $R_{7a}$, $R_{8a}$ and R $_{11a}$ are each hydrogen

i) $R_{2a}$ is not methoxy when $R_{1a}$, $R_{3a}$ and $R_{4a}$ are each hydrogen,

ii) $R_{2a}$ and $R_{3a}$ are not both methoxy when $R_{1a}$and $R_{4a}$ are both hydrogen,

iii) $R_{1a}$ and $R_{4a}$ are not both methoxy when $R_{2a}$and $R_{3a}$ are both hydrogen, and

b) when $R_{1a}$, $R_{3a}$ , $R_{4a}$, $R_{5a}$, $R_{6a}$, $R_{7a}$ and $R_{11a}$ are each hydrogen and $R_{9a}$ and $R_{10a}$ are both methyl $R_{2a}$ is not methoxy when R $_{8a}$ is methyl,

c) when $R_1$, $R_4$, $R_7$, $R_8$ and $R_{11}$ are hydrogen, $R_9$ and $R_{10}$ are both ethyl, $R_2$ and $R_3$ are both methoxy and one of $R_5$ or $R_6$ is hydrogen the remainder of $R_5$ or $R_6$ is not hydrogen or methyl, and pharmaceutically acceptable salts thereof,

b)

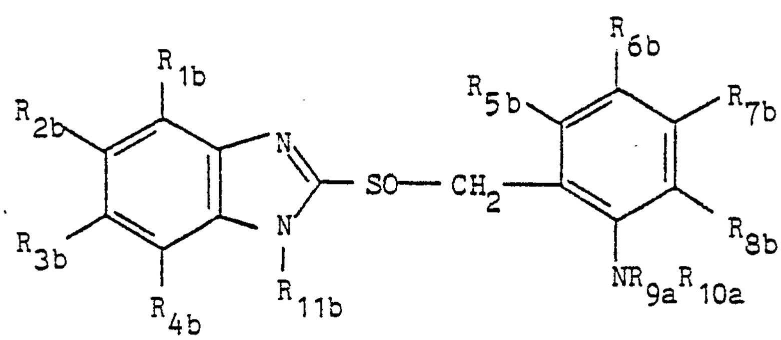

Ib

in which at least one of $R_{5b}$ , $R_{6b}$, $R_{7b}$ and $R_{8b}$ is alkyl C I to C6, and the remainder, which may be the same or different, are each hydrogen, halogen, alkoxy C I to C6, $-NO_2$ or $-NR_{13}R_{14}$,

$R_{1b}$, $R_{2b}$, $R_{3b}$ and $R_{4b}$ , which may be the same or different, are each hydrogen, alkoxy C I to C6, alkyl C I to C6, $-NR_{13}R_{14}$, $-NO_2$ or COOH or an ester thereof,

$R_{9a}$, $R_{10a}$, $R_{13}$ and $R_{14}$ are as defined above,

$R_{11b}$ is hydrogen, or alkyl C I to C6 optionally substituted by phenyl,

provided that

a) when $R_{11b}$ is hydrogen, then at least one of $R_{1b}$, R $_{2b}$, $R_{3b}$ and $R_{4b}$ is other than hydrogen;

b) when $R_{1b}$, $R_{3b}$, $R_{4b}$, $R_{5b}$, $R_{6b}$, $R_{7b}$ and $R_{11b}$ are each hydrogen, $R_{9a}$ and $R_{10a}$are both methyl, and $R_{8b}$ is methyl then $R_{2b}$ is not methoxy,

c) when $R_{1b}$, $R_{4b}$, $R_{7b}$, $R_{8b}$ and $R_{11b}$ are each hydrogen, $R_{9a}$ and $R_{10a}$ are both ethyl, $R_{2b}$ and $R_{3b}$are both methoxy and one of $R_{5b}$ or $R_{6b}$ is hydrogen the remainder of $R_{5b}$ or $R_{6b}$ is not methyl, and pharmaceutically acceptable salts thereof,

c)

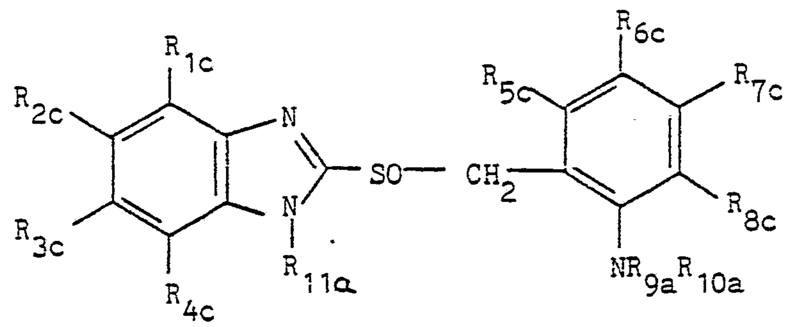

Ic

in which $R_{9a}$ and $R_{10a}$ which are as defined above, together comprise more than 3 carbon atoms,

$R_{1c}$, $R_{2c}$, $R_{3c}$, $R_{4c}$, $R_{5c}$, $R_{6c}$, $R_{7c}$ and $R_{8c}$, which may be the same or different, are each hydrogen, halogen, alkoxy C l to C6 or alkyl C l to C6,

$R_{11a}$ is as defined above,

provided that

a) when $R_{11a}$ is hydrogen, then at least one of $R_{1c}$, $R_{2c}$ $R_{3c}$ and $R_{4c}$ is other than hydrogen,

b) when $R_{1c}$, $R_{4c}$, $R_{7c}$, $R_{8c}$ and $R_{11a}$ are each hydrogen, $R_{9a}$ and $R_{10a}$ are both ethyl, $R_{2c}$ and $R_{3c}$are both methoxy and one of $R_{5c}$ or $R_{6c}$ is hydrogen the remainder of $R_{5c}$ or $R_{6c}$is not hydrogen or methyl, and pharmaceutically acceptable salts thereof,

d)

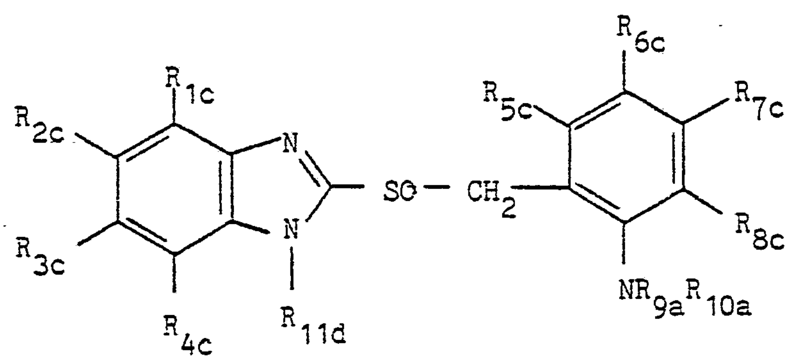

Id

in which $R_{11d}$ is alkenyl C2 to C6 or alkyl C l to C6 optionally substituted by phenyl,

$R_{1c}$, $R_{2c}$, $R_{3c}$, $R_{4c}$, $R_{5c}$, $R_{6c}$, $R_{7c}$, $R_{8c}$, $R_{9a}$ and $R_{10a}$ are as defined above,

provided that when $R_{1c}$, $R_{2c}$, $R_{3c}$, $R_{4c}$, $R_{5c}$, $R_{6c}$, $R_{7c}$ and $R_{8c}$ are each hydrogen and $R_{9a}$ and $R_{10a}$ are both methyl that $R_{11d}$ is not methyl,

and pharmaceutically acceptable salts therefore.

According to our invention we also provide a pharmaceutical composition comprising (preferably a minor proportion of) a compound of formula I, or a pharmaceutically acceptable salt thereof, as active ingredient, in admixture with a pharmaceutically acceptable adjuvant, diluent or carrier. Examples of suitable adjuvants, diluents or carriers are:-for tablets and dragees; lactose, starch, talc or stearic acid; for capsules, tartaric acid or lactose; for suppositories, natural or hardened oils or wax; and for injections (i.m. or i.v.) or enemas water, surfactants and preservatives. The compounds may also be administered transdermally, eg in an ointment base. The compound of formula I, or the pharmaceutically acceptable salt thereof, preferably has a mass median diameter of from 0.0l to l0 microns. The compound of such particle size may be made

by grinding or milling followed if necessary by particle size classification using, for example, a sieve. The compositions may also contain suitable preserving, stabilising, and wetting agents, solubilizers, sweetening and colouring agents and flavourings. The compositions may, if desired, be formulated in sustained release form.

The compounds may, if desired, be co-administered, with (eg as a mixture with) an antacid buffer.

We prefer compositions which are designed to be taken by ingestion or rectally and to release their contents in the intestine. We particularly prefer compositions which will pass through the acidic parts of the gastrointestinal tract unaffected, eg enteric coated formulations.

The compounds of formula I are optically active and may be resolved into their optical isomers using conventional techniques known per se. The invention therefore provides the compounds as their optical isomers, or as mixtures, eg racemic mixtures, thereof.

The compounds of formula I may exist in tautomeric forms and these tautomeric forms are included in the definition of the compounds of formula I.

The invention is illustrated, but in no way limited by the following Examples in which temperatures are in degrees centigrade.

Example I

2-(5,6-Diethoxy-IH-benzimidazol-2-yl sulphinyl methyl)-N,N-diethyl benzenamine

a) I,2-Diethoxy benzene

I,2-dihydroxybenzene (22g, 0.2mmole) in dimethyl formamide (300ml) was treated with $K_2CO_3$ - (0.5mmole, 69g) under $N_2$, then iodoethane (0.5mmole, 78g, 40ml) was added and the whole was stirred at 25° overnight under $N_2$. The reaction was poured into water (I.5L), acidified and ether extracted. The organic phase was washed with water (×5), dried and evaporated to afford the sub-title compound as a crystalline solid, mp 4I-2°.

b) I,2-Diethoxy-4,5-dinitrobenzene

The product of step a) above (50g) was added over about 7 minutes to 60% aqueous nitric acid (437ml) with stirring. The temperature rose rapidly and a yellow solid formed. The mixture was stirred for I I/2 hours and then poured into ice/water (2L) and filtered. The solid was slurried with aqueous sodium bicarbonate, filtered, washed with water (II) and recrystallised from ethanol to give the sub-title compound, mp I04-6°.

c) 5,6-Diethoxy-IH-benzimidazole-2-thiol

The product of step b) above (38g, 0.I48mmole) in dry dimethylformamide (600ml) was hydrogenated at 3 atmospheres pressure over I0% Pd-C (3.8g). The catalyst was filtered off under $N_2$. The filtrate was treated with $CS_2$ (I00ml, I26g, I.65mmole), and heated under $N_2$ at 65° for I8 hours then distilled in vacuo. The solid residue was slurried with ether to afford the sub-title compound as a grey crystalline powder, mp greater than 250°, MS $M^+$ 238.

d) 2-N,N-Diethylaminobenzaldehyde

A mixture of 2-fluorobenzaldehyde (24.7g), potassium carbonate (34.5g), diethylamine (45ml) and N-methylpyrrolidone (90ml) was heated, with stirring, at I20° for 40 hours. The mixture was poured into water, extracted with ether and the combined organic extracts washed with water, dried and concentrated to give a dark liquid. This liquid was distilled under reduced pressure to give 27g of 2-N,N-diethylaminobenzaldehyde (bp 90° at I.5 mbar).

e) 2-(Diethylamino)-benzene methanol)

The product of step d) above (4.5g, 25.4mmole) in ethanol (I00ml) was treated with $NaBH_4$ (30mmole, I.I4g) at 25° overnight. Dilute HCl (20ml) was added, and I5 minutes later, the ethanol was evaporated and the residue was basified and extracted in ethyl acetate to yield the sub-title compound. MS $M^+$ I79.

f) 2-(5,6-Diethoxy-IH-benzimidazol-2-yl thiomethyl)-N,N-diethyl benzenamine

The product of step e) above (7.5g, 4I.8mmole) in $CH_2Cl_2$ (I00ml) was cooled in ice and treated with $SOCl_2$ (60mmole, 7.I4g, 4.38ml). The cooling bath was removed and the mixture was left at 25° overnight, then distilled in vacuo at 35°. The residue was dissolved in dry dimethylformamide (I20ml). Half of this solution was added to a mixture of the product of step c) above (4.879g, 20mmole) in dry dimethylformamide (25ml) and $K_2CO_3$ (50mmole, 6.9g). This mixture was stirred at 25° for 48 hours, poured into water (800ml) and extracted into ethyl acetate. The extract was washed well with water and dried. Flash chromatography ($SO_2$/4: I $CH_2Cl_2$-ethyl acetate) gave the sub-title compound as a cream solid, mp 78-82°.

g) 2-(5,6-Diethoxy-lH-benzimidazol-2-yl sulphinylmethyl)-N,N-diethyl benzenamine

The product of step f) above (6.5g, l6.29mmole) in $CH_2Cl_2$ (l50ml) was cooled to 0° and m-chloroperbenzoic acid (l6.29mmole = 2.95g 95%) was added. The reaction was stirred for 2 hours in ice then at 25° for 2 hours, washed with aqueous $NaHSO_3$, then aqueous $NaHCO_3$, then water. The reaction mixture was dried, evaporated and purified by flash chromatography ($SO_2$/3:l $CH_2Cl_2$-ethyl acetate) to give the major 5 product as a gum (4.6g). Trituration with ether gave the title compound as a white powder, mp ll6-7°.

The compounds of Examples 2 to 42 may be prepared by the method described in Example l using the appropriate starting materials.

Example 2

a) 2-(4,5-Dimethoxy-lH-benzimidazol-2-yl thio methyl)-N,N-dimethyl benzenamine , mp 97-99°.

b) 2-(4,5-Dimethoxy-lH-benzimidazol-2-yl sulphinyl methyl)-N,N-dimethyl benzenamine, mp l0l-2° (softens).

Example 3

a) 2-(4,7-Dimethoxy-lH-benzimidazol-2-yl thio methyl)-N,N-4-trimethyl benzenamine, MS $M^+$ 357.

b) 2-(4,7-Dimethoxy-lH-benzimidazol-2-yl sulphinyl methyl)-N,N-4-trimethyl benzenamine, mp l29-l30°.

Example 4

a) 2-(4,5,6-Trimethoxy-lH-benzimidazol-2-yl thio methyl)-N,N-dimethyl benzenamine , MS $M^+$ 387.

b) 2-(4,5,6-Trimethoxy-lH-benzimidazol-2-yl sulphinyl methyl)-N,N-dimethyl benzenamine, mp l25-7°.

Example 5

a) 2-(4,6-Dimethoxy-lH-benzimidazol-2-yl thio methyl)-N,N-dimethyl benzenamine , MS $M^+$ 343.

b) 2-(4,6-Dimethoxy-lH-benzimidazol-2-yl sulphinyl methyl)-N,N-dimethyl benzenamine, mp 70°.

Example 6

a) Methyl 6-methoxy-2-(2-dimethylamino-phenylmethyl thio)-lH-benzimidazol-5-carboxylate , mp 56°.

b) Methyl 6-methoxy-2-(2-dimethylamino-phenylmethyl sulphinyl)-lH-benzimidazol-5-carboxylate, mp 6l°.

Example 7

a) 2-(5-Methoxy-lH-benzimidazol-2-yl thio methyl)-N,N-dimethyl-4-(l,l-dimethylethyl)benzenamine, mp 57-59°.

b) 2-(5-Methoxy-lH-benzimidazol-2-yl sulphinyl methyl)-N,N-dimethyl-4-(l,l-dimethylethyl)-benzenamine, mp 70-l°.

Example 8

a) 2-(5,6-Dimethoxy-lH-benzimidazol-2-yl thio methyl)-5-nitro-N,N-dimethyl benzenamine, MS $M^+$ 388.

b) 2-(5,6-Dimethoxy-lH-benzimidazol-2-yl sulphinyl methyl)-5-nitro-N,N-dimethyl benzenamine, mp l72-4°.

Example 9

a) 2-(5,6-Dimethoxy-lH-benzimidazol-2-yl thio methyl)-N,N,4-trimethyl benzenamine, mp l25°.
b) 2-(5,6-Dimethoxy-lH-benzimidazol-2-yl sulphinyl methyl)-N,N,4-trimethyl benzenamine, mp l36-8°.

Example l0

a) 2-(5,6-Dimethoxy-lH-benzimidazol-2-yl thio methyl)-N,N-dimethyl-4-(l,l-dimethylethyl)benzenamine, mp 77-79°.
b) 2-(5,6-Dimethoxy-lH-benzimidazol-2-yl sulphinyl methyl)-N,N-dimethyl-4-(l,l-dimethylethyl)-benzenamine, mp 87-9°.

Example ll

a) 2-(5,6-Diethoxy-lH-benzimidazol-2-yl thio methyl)-N,N-dimethyl benzenamine , mp l09-ll0°.
b) 2-(5,6-Diethoxy-lH-benzimidazol-2-yl sulphinyl methyl)-N,N-dimethyl benzenamine, mp ll0° (d).

Example l2

a) 2-(5,6-Diethoxy-lH-benzimidazol-2-yl thio methyl)-N-ethyl-N-methyl benzenamine, MS $M^+$ 385.
b) 2-(5,6-Diethoxy-lH-benzimidazol-2-yl sulphinyl methyl)-N-ethyl-N-methyl benzenamine, mp l05-7°.

Example l3

a) 2-(5-Butyloxy-6-ethoxy-lH-benzimidazol-2-yl thio methyl)-N,N-dimethyl benzenamine, MS $M^+$ 399.
b) 2-(5-Butyloxy-6-ethoxy-lH-benzimidazol-2-yl sulphinyl methyl)-N,N-dimethyl benzenamine, mp 87-97° (d).

Example l4

a) 2-(4,5,7-Trimethoxy-lH-benzimidazol-2-yl thio methyl)-N,N-dimethyl benzenamine, mp 96-98°.
b) 2-(4,5,7-Trimethoxy-lH-benzimidazol-2-yl sulphinyl methyl)-N,N-dimethyl benzenamine, mp ll6°.

Example l5

a) 2-(5,6-Diethoxy-lH-benzimidazol-2-yl thio methyl)-N,N,4-trimethyl benzenamine, mp 89-9l°.
b) 2-(5,6-Diethoxy-lH-benzimidazol-2-yl sulphinyl methyl)-N,N,4-trimethyl benzenamine, mp l38° (d).

Example l6

a) 2-(5-Methoxy-lH-benzimidazol-2-yl thio methyl)-N,N,3-trimethyl benzenamine, mp l2l-l23°.
b) 2-(5-Methoxy-lH-benzimidazol-2-yl sulphinyl methyl)-N,N,3-trimethyl benzenamine, mp 66° (d).

Example l7

a) 2-(5,6-Diethoxy-lH-benzimidazol-2-yl thio methyl)-N,N,3-trimethyl benzenamine, mp l00-l0l°.
b) 2-(5,6-Diethoxy-lH-benzimidazol-2-yl sulphinyl methyl)-N,N,3-trimethyl benzenamine, mp l50-l°.

Example 18

a) 2-(5,6-Dimethoxy-1H-benzimidazol-2-yl thio methyl)-N,N,3-trimethyl benzenamine, mp 69-72°.
b) 2-(5,6-Dimethoxy-1H-benzimidazol-2-yl sulphinyl methyl)-N,N,3-trimethyl benzenamine, mp 144-5°.

Example 19

a) 2-(5,6-Diethoxy-1H-benzimidazol-2-yl thio methyl)-4-methoxy-N,N,3,5-tetramethyl benzenamine, mp 64° (d).
b) 2-(5,6-Diethoxy-1H-benzimidazol-2-yl sulphinyl methyl)-4-methoxy-N,N,3,5-tetramethyl benzenamine, mp 132-4°(d).

Example 20

a) 2-(4,7-Dimethoxy-1H-benzimidazol-2-yl thio methyl)-N-cyclohexyl-N-methyl benzenamine, mp 113-114°.
b) 2-(4,7-Dimethoxy-1H-benzimidazol-2-yl sulphinyl methyl)-N-cyclohexyl-N-methyl benzenamine, mp 74° (d).

Example 21

a) 2-(5-Methoxy-1H-benzimidazol-2-yl thio methyl)-N,N-diethyl benzenamine, MS $M^+$ 341.
b) 2-(5-Methoxy-1H-benzimidazol-2-yl sulphinyl methyl)-N,N-diethyl benzenamine , MS (FAB) m/e 358 (M+1).

Example 22

a) 2-(5-Methoxy-1H-benzimidazol-2-yl thiomethyl)-N-cyclohexyl-N-methyl benzenamine, mp 62-64°.
b) 2-(5-Methoxy-1H-benzimidazol-2-yl sulphinylmethyl)-N-cyclohexyl-N-methyl benzenamine, mp 71° (d).

Example 23

a) 2-(4-Methoxy-1H-benzimidazol-2-yl thio methyl)-N,N-dimethyl benzenamine, mp 100°.
b) 2-(4-Methoxy-1H-benzimidazol-2-yl sulphinyl methyl)-N,N-dimethyl benzenamine
CHN Analysis:
Found: C,61.68; H,5.7; N,12.66; S,9.53
$C_{17}H_{19}N_3O_2S$
Required: C,62.0; H,5.78; N,12.8; S,9.73%

Example 24

a) 2-(4,7-Diethoxy-1H-benzimidazol-2-yl thio methyl)-N,N-dimethyl benzenamine , MS $M^+$ 371.
b) 2-(4,7-Diethoxy-1H-benzimidazol-2-yl sulphinyl methyl)-N,N-dimethyl benzenamine, mp 83-6°.

Example 25

a) 2-(5,6-Diethoxy-2-1H-benzimidazolylthiomethyl)-N,N-diethyl-3-methyl-benzenamine , mp 54°.
b) 2-(5,6-Diethoxy-2-1H-benzimidazolylsulphinylmethyl)-N,N-diethyl-3-methyl-benzenamine , mp 49°.

Example 26

a) 2-(5,6-Diethoxy-2-lH-benzimidazolylthiomethyl)-N,N,5-trimethyl-benzenamine , mp l05°.
b) 2-(5,6-Diethoxy-2-lH-benzimidazoiylsulphinylmethyl)-N,N,5-trimethyl-benzenamine , mp l36°.

Example 27

a) 2-(5-Methoxy-6-propyloxy-lH-benzimidazol-2-yl thiomethyl)-N,N-dimethyl benzenamine, mp l07°.
b) 2-(5-Methoxy-6-propyloxy-lH-benzimidazol-2-yl sulphinylmethyl)-N,N-dimethyl benzenamine, mp l38-l40°.

Example 28

a) 2-(5,6-Diethoxy-lH-benzimidazol-2-yl)thiomethyl)-N,N-diethyl-5-methyl-benzenamine , mp 92-94°.
b) 2-(5,6-Diethoxy-lH-benzimidazol-2-yl sulphinylmethyl)-N,N-diethyl-5-methyl-benzenamine, mp l20-2°.

Example 29

a) 5,6-Diethoxy-2-[2-methyl-6-(l-piperidyl)phenylmethylthio]-lH-benzimidazole , mp l48-l49°.
b) 5,6-Diethoxy-2-[2-methyl-6-(l-piperidyl)phenylmethylsulphinyl]-lH-benzimidazole , mp 80°.

Example 30

a) 2-(5,6-Diethoxy-lH-2-benzimidazolylthiomethyl)-N,N,3,6-tetramethyl-benzenamine , MS $M^+$ 399.
b) 2-(5,6-Diethoxy-lH-2-benzimidazolylsulphinylmethyl)-N,N,3,6-tetramethyl-benzenamine , mp l6l-2°.

Example 3l

a) 2-(5-Methoxy-lH-benzimidazol-2-yl thiomethyl)-4-methoxy-N,N,6-trimethyl benzenamine, MS $M^+$ 357.

b) 2-(5-Methoxy-lH-benzimidazol-2-yl sulphinylmethyl)-4-methoxy-N,N,6-trimethyl benzenamine, mp 73°.

Example 32

a) 2-(5-Chloro-lH-benzimidazol-2-yl thiomethyl)-N,N-diethyl benzenamine, MS $M^{-1}$ 345/347.
b) 2-(5-Chloro-lH-benzimidazol-2-yl sulphinylmethyl)-N,N-diethyl benzenamine, mp 9l-4°.

Example 33

a) 2-(5-Hydroxy-lH-benzimidazol-2-yl thiomethyl)-N,N-dimethyl benzenamine,
Nmr ($CDCl_3$) delta 7.2 (m,6H), 6.8 (d.o.d.,lH), 4.2l (broad,s,2H), 2.73 (broad,s,6H) + l/2mmole $(C_2H_5)_2O$.
b) 2-(5-Hydroxy-lH-benzimidazol-2-yl sulphinylmethyl)-N,N-dimethyl benzenamine , mp l66° (dec).

Example 34

a) N,N-Dimethyl-2-(5-(4-morpholinyl, 4-oxide)-ethoxy]-lH-benzimidazol-2-yl thiomethyl)benzenamine,
Nmr ($CDCl_3$) delta 7.48 (d,ll/2H), 7.25 (m,lH), 7.l5 (m,3H), 6.80 (m,ll/2H), 4.36 (s,2H), 4.ll (t,2H), 3.74 (t,4H), 2.9l (s,6H), 2.8l (t,2H), 2.58 (broad,t,4H).

b) N,N-Dimethyl-2-(5-(4-morpholinyl, 4-oxide)-ethoxy]-IH-benzimidazol-2-yl sulphinylmethyl)-benzenamine
CHN Analysis for I/2 $H_2O$
Theory (Found): C,58.26 (58.37); H,6.44 (6.23); N,I2.37 (I2.37); S,7.07 (7.45).

Example 35

a) N,N-Dimethyl-2-(5-hydroxy-6-methoxy-IH-benzimidazol-2-yl thiomethyl)benzenamine,
Nmr (DMSO) delta I2.23 and I2.I3 (broad,IH), 8.76 and 8.56 (broad,IH), 7.36 (d,IH), 7.23 (t,IH), 7.I7 (d,IH), 7.09 (s,I/2H), 7.00 (t,IH), 6.92 (broad,s,I/2H), 6.85 (broad,s,I/2H), 6.76 (s,I/2H), 4.53 (s,2H), 3.77 (s,3H), 2.66 (s,6H).
b) N,N-Dimethyl-2-(5-hydroxy-6-methoxy-IH-benzimidazol-2-yl sulphinylmethyl)benzenamine
CHN Analysis:
Theory (Found): C,59.I3 (59.I6); H,5.55 (5.78); N,I2.I7 (II.I4); S,9.28 (9.I4).

Example 36

a) 2-(5,6-Dihydroxy-IH-benzimidazol-2-yl thio methyl)-N,N-dimethyl benzenamine ,
Nmr (DMSO) delta 8.5 (broad,s,2H), 7.I (m,4H), 6.83 (s,2H), 4.54 (s,2H), 2.68 (s,6H).
b) 2-(5,6-Dihydroxy-IH-benzimidazol-2-yl sulphinyl methyl)-N,N-dimethyl benzenamine,
CHN Analysis for I/2 $H_2O$
Theory (Found): C,56.45 (56.47); H,5.33 (5.27); N,I2.34 (I2.4I); S,9.42 (9.63).

Example 37

a) 2-(5-Hydroxymethyl-IH-benzimidazol-2-yl thio methyl)-N,N-dimethyl benzenamine , mp 70°.
b) 2-(5-Hydroxymethyl-IH-benzimidazol-2-yl sulphinyl methyl)-N,N-dimethyl benzenamine, mp 60°.

Example 38

a) 2-(5-Hydroxymethyl-6-methoxy-IH-benzimidazol-2-yl thiomethyl)-N,N-dimethyl benzenamine, mp 68°.
b) 2-(5-Hydroxymethyl-6-methoxy-IH-benzimidazol-2-yl sulphinylmethyl)-N,N-dimethyl benzenamine, mp 75°.

Example 39

a) 2-(6,7-Dihydrodioxino[2,3-f]-IH-benzimidazol-2-yl thiomethyl)-N,N-dimethyl benzenamine, I50-I5I°.
b) 2-(6,7-Dihydrodioxino[2,3-f]-IH-benzimidazol-2-yl sulphinylmethyl)-N,N-dimethyl benzenamine,
Nmr ($CDCl_3$) delta II.I (broad, IH), 7.0 (m,6H), 4.82 (d,IH), 4.46 (d,IH), 4.3I (s,4H), 2.65 (s,6H).

Example 40

a) 2-(7,8-Dihydrodioxino[2,3-e]-IH-benzimidazol-2-yl thiomethyl)-N,N-dimethyl benzenamine,
Nmr ($CDCl_3$) delta II.6 (broad,IH), 7.2 (m,4H), 6.78 (d,IH), 6.73 (d,IH), 4.38 and 4.35 (s,6H), 2.90 (s,6H).
b) 2-(7,8-Dihydrodioxino[2,3-e]-IH-benzimidazol-2-yl sulphinylmethyl)-N,N-dimethyl benzenamine,
Nmr ($CDCl_3$) delta II.2 (broad, IH), 7.0 (m,6H), 4.88 (d,IH), 4.46 (d,IH), 4.37 (s,4H), 2.66 (s,6H) + I/4 mmole ethyl acetate.

Example 4l

a) 2-(2,3-Dihydro-2-oxo-5H-imadazo[4,5-f]benzoxazol-6-yl thiomethyl)-N,N-dimethyl benzenamine
CHN Analysis:
Theory (Found): C,59.36 (59.l8); H,4.49 (4.88); N,l6.29 (l5.92); S,9.92 (8.82); $H_2O$,l.03 (l.03).

b) 2-(2,3-Dihydro-2-oxo-5H-imadazo[4,5-f]benzoxazol-6-yl sulphinylmethyl)-N,N-dimethyl benzenamine
CHN Analysis:
Theory (Found): C,57.29 (57.23); H,4.53 (4.62); N,l5.72 (l5.42); S,8.99 (8.63).

Example 42

2-(4,7-Dimethoxy-l-methyl-lH-benzimidazol-2-yl sulphinylmethyl)-N,N-dimethyl benzenamine

A solution of 2-(4-7-dimethoxy-lH-benzimidazol-2-yl sulphinylmethyl)-N,N-dimethyl benzenamine (lg, Example l6, GB 2,l6l,l60) and iodomethane (0.l9ml) in dry dimethylformamide (20ml) containing anhydrous potassium carbonate (0.8g) was stirred at 25° for 6 hours. The mixture was quenched with water. The organic phase was washed with brine, dried over magnesium sulphate, filtered and evaporated to leave a yellow oil which was purified by flash chromatography eluting with dichloromethane/ethyl acetate (5:l). The required fractions were evaporated to leave a yellow oil which solidified on standing. The solid was triturated with pentane, filtered and dried under vacuum to yield the title compound (0.75g) mp 94-95°.

By the method of Example 42 and using the appropriate starting materials may be prepared the compounds of Examples 43 - 55.

Example 43 2-(5,6-Dimethoxy-l-methyl-lH-benzimidazol-2-yl sulphinylmethyl)-N,N-dimethyl benzenamine, mp ll3-4°.

Example 44 2-(5,6-Dimethoxy-l-propyl-lH-benzimidazol-2-yl sulphinylmethyl)-N,N-dimethyl benzenamine, MS (Fast Atom Bombardment) m/e 402 (M+l).

Example 45 2-(5,6-Dimethoxy-l-phenylmethyl-lH-benzimidazol-2-yl sulphinylmethyl)-N,N-dimethyl benzenamine, mp 52°.

Example 46 2-(5-Methoxy-l-methyl-lH-benzimidazol-2-yl sulphinyl methyl)-N,N-dimethyl benzenamine, MS (FAB) m/e 344 (M+l).

Example 47 2-(5,6-Diethoxy-l-methyl-lH-benzimidazol-2-yl sulphinyl methyl)-N,N-diethyl benzenamine, mp 86-7°.

Example 48 N,N,-Diethyl-2-(5,6-dimethoxy-l-methyl-lH-benzimidazol-2-yl sulphinylmethyl)benzenamine, mp 96-8°.

Example 49 2-(5,6-Dimethoxy-l-phenyl-lH-benzimidazol-2-yl sulphinylmethyl)-N,N,-dimethyl benzenamine, mp ll5°.

Example 50 l-Methyl-2-[2-(l-piperidyl)phenylmethylsulphinyl]-lH-benzimidazole, mp l36-7°.

Example 5l 2-(l-Methyl-lH-benzimidazol-2-yl sulphinylmethyl)-N-ethyl-N-propyl benzenamine , MS M+ 355.

Example 52 2-(l-Prop-2-enyl-lH-benzimidazol-2-yl sulphinyl methyl)-N,N-dimethyl benzenamine, MS (FAB) m/e 340 (M+l).

Example 53 2-(l-Methyl-lH-benzimidazol-2-yl sulphinylmethyl)-4-fluoro-N,N-dimethyl benzenamine, MS (FAB) m/e 335 (M+l).

Example 54 N,N,6-Trimethyl-2-(l-methyl-lH-benzimidazol-2-yl sulphinylmethyl)-benzenamine , mp 89-9l°.

Example 55 2-(l-Methyl-6,7-dihydrodioxino[2,3-f]-lH-benzimidazol-2-yl sulphinylmethyl)-N,N-dimethyl benzenamine, mp l05-l08°.

## Claims

l. A compound of formula I,

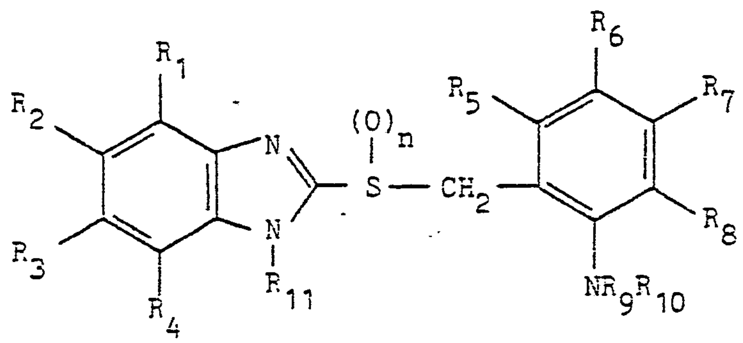

I

in which $R_1$, $R_2$, $R_3$, $R_4$, $R_5$, $R_6$, $R_7$ and $R_8$, which may be the same or different, are each hydrogen, halogen, alkyl C l to C6, $-(CH_2)_mOH$, $-NO_2$, $-NR_{13}R_{14}$, -COOH or an ester thereof, or alkoxy C l to C6 optionally substituted by a saturated heterocyclic ring,

or an adjacent pair of $R_1$, $R_2$, $R_3$ and $R_4$ may, in addition to the values given above, form an $-OCH_2CH_2O-$or -OCONH-chain,

m is 0 or l,

n is 0 or l,

$R_{13}$ and $R_{14}$, which may be the same or different, are each hydrogen or alkyl C l to C6,

$R_{11}$ is hydrogen, phenyl, alkenyl C2 to C6, or alkyl C l to C6 optionally substituted by phenyl,

$R_9$ and $R_{10}$, which may be the same or different, are each cycloalkyl C3 to C7 or alkyl C l to C6, optionally substituted by phenyl, or

$R_9$ and $R_{10}$, together with the nitrogen atom to which they are attached, may form a saturated 6 to 8 inclusive membered ring which contains no heteroatoms other than the nitrogen atom to which $R_9$ and $R_{10}$ are attached,

provided that

a) when $R_{11}$ is hydrogen, then at least one of $R_1$, $R_2$, $R_3$, $R_4$ is other than hydrogen;

b) when $R_5$, $R_6$, $R_{11}$ are each hydrogen and $R_9$, $R_{10}$ are both methyl, then

i) when $R_1$, $R_3$, $R_4$, $R_7$, $R_8$ are each hydrogen, $R_2$ is not chloro, methoxycarbonyl, methyl, methoxy, $-NO_2$ or $-NH_2$;

ii) when $R_1$, $R_4$, $R_7$, $R_8$ are each hydrogen, $R_2$ and $R_3$ do not both represent methyl, chloro or methoxy;

iii) when $R_2$, $R_3$, $R_7$, $R_8$ are each hydrogen, $R_1$ and $R_4$ do not both represent methoxy;
iv) when $R_2$, $R_3$, $R_4$, $R_7$, $R_8$ are each hydrogen, $R_1$ is not methyl;
v) when $R_1$, $R_8$, $R_4$, $R_7$ are each hydrogen and $R_3$ is methyl, $R_2$ is not methoxy;
vi) when $R_1$, $R_3$, $R_4$, $R_8$ are each hygrogen and $R_7$ is methoxy, $R_2$ is not chloro;
c) when $R_1$ to $R_8$ each represent hydrogen, $R_9$, $R_{10}$, $R_{11}$ do not each represent methyl;
d) when $R_1$, $R_4$, $R_7$, $R_8$ and $R_{11}$ are each hydrogen, $R_9$ and $R_{10}$ are both ethyl, $R_2$ and $R_3$ are both methoxy and one of $R_5$ or $R_6$ is hydrogen the remainder of $R_5$ or $R_6$ is not hydrogen or methyl,
and pharmaceutically acceptable salts thereof.

2. A compound according to Claim I, in which n is I and at least two of $R_1$ to $R_4$ are alkoxy C I to C6 and $R_9$ and $R_{10}$, which may be the same or different, are alkyl C I to C6 or together comprise more than 3 carbon atoms.

3. A compound according to Claim I or Claim 2, in which n is I and at least two of $R_1$ to $R_4$ are selected from methoxy or ethoxy and $R_9$ and $R_{10}$, which may be the same or different, are methyl, ethyl or together comprise more than 3 carbon atoms.

4. 2-(5,6-Diethoxy-IH-benzimidazol-2-yl sulphinylmethyl)-N,N-diethyl benzenamine, or a pharmaceutically acceptable salt thereof.

5. 2-(5,6-Diethoxy-IH-benzimidazol-2-yl thiomethyl)-N,N-diethyl benzenamine
2-(4,5-Dimethoxy-IH-benzimidazol-2-yl thio methyl)-N,N-dimethyl benzenamine
2-(4,5-Dimethoxy-IH-benzimidazol-2-yl sulphinyl methyl)-N,N-dimethyl benzenamine
2-(4,7-Dimethoxy-IH-benzimidazol-2-yl thio methyl)-N,N-4-trimethyl benzenamine
2-(4,7-Dimethoxy-IH-benzimidazol-2-yl sulphinyl methyl)-N,N-4-trimethyl benzenamine
2-(4,5,6-Trimethoxy-IH-benzimidazol-2-yl thio methyl)-N,N-dimethyl benzenamine
2-(4,5,6-Trimethoxy-IH-benzimidazol-2-yl sulphinyl methyl)-N,N-dimethyl benzenamine
2-(4,6-Dimethoxy-IH-benzimidazol-2-yl thio methyl)-N,N-dimethyl benzenamine
2-(4,6-Dimethoxy-IH-benzimidazol-2-yl sulphinyl methyl)-N,N-dimethyl benzenamine
Methyl 6-methoxy-2-(2-dimethylamino-phenylmethyl thio)-IH-benzimidazol-5-carboxylate
Methyl 6-methoxy-2-(2-dimethylamino-phenylmethyl sulphinyl)-IH-benzimidazol-5-carboxylate
2-(5-Methoxy-IH-benzimidazol-2-yl thio methyl)-N,N-dimethyl-4-(I,I-dimethylethyl)benzenamine
2-(5-Methoxy-IH-benzimidazol-2-yl sulphinyl methyl)-N,N-dimethyl-4-(I,I-dimethylethyl)benzenamine
2-(5,6-Dimethoxy-IH-benzimidazol-2-yl thio methyl)-5-nitro-N,N-dimethyl benzenamine
2-(5,6-Dimethoxy-IH-benzimidazol-2-yl sulphinyl methyl)-5-nitro-N,N-dimethyl benzenamine
2-(5,6-Dimethoxy-IH-benzimidazol-2-yl thio methyl)-N,N,4-trimethyl benzenamine
2-(5,6-Dimethoxy-IH-benzimidazol-2-yl sulphinyl methyl)-N,N,4-trimethyl benzenamine
2-(5,6-Dimethoxy-IH-benzimidazol-2-yl thio methyl)-N,N-dimethyl-4-(I,I-dimethylethyl)benzenamine
2-(5,6-Dimethoxy-IH-benzimidazol-2-yl sulphinyl methyl)-N,N-dimethyl-4-(I,I-dimethylethyl)benzenamine
2-(5,6-Diethoxy-IH-benzimidazol-2-yl thio methyl)-N,N-dimethyl benzenamine
2-(5,6-Diethoxy-IH-benzimidazol-2-yl sulphinyl methyl)-N,N-dimethyl benzenamine
2-(5,6-Diethoxy-IH-benzimidazol-2-yl thio methyl)-N-ethyl-N-methyl benzenamine
2-(5,6-Diethoxy-IH-benzimidazol-2-yl sulphinyl methyl)-N-ethyl-N-methyl benzenamine
2-(5-Butyloxy-6-ethoxy-IH-benzimidazol-2-yl thio methyl)-N,N-dimethyl benzenamine
2-(5-Butyloxy-6-ethoxy-IH-benzimidazol-2-yl sulphinyl methyl)-N,N-dimethyl benzenamine
2-(4,5,7-Trimethoxy-IH-benzimidazol-2-yl thio methyl)-N,N-dimethyl benzenamine
2-(4,5,7-Trimethoxy-IH-benzimidazol-2-yl sulphinyl methyl)-N,N-dimethyl benzenamine
2-(5,6-Diethoxy-IH-benzimidazol-2-yl thio methyl)-N,N,4-trimethyl benzenamine
2-(5,6-Diethoxy-IH-benzimidazol-2-yl sulphinyl methyl)-N,N,4-trimethyl benzenamine
2-(5-Methoxy-IH-benzimidazol-2-yl thio methyl)-N,N,3-trimethyl benzenamine
2-(5-Methoxy-IH-benzimidazol-2-yl sulphinyl methyl)-N,N,3-trimethyl benzenamine
2-(5,6-Diethoxy-IH-benzimidazol-2-yl thio methyl)-N,N,3-trimethyl benzenamine
2-(5,6-Diethoxy-IH-benzimidazol-2-yl sulphinyl methyl)-N,N,3-trimethyl benzenamine
2-(5,6-Dimethoxy-IH-benzimidazol-2-yl thio methyl)-N,N,3-trimethyl benzenamine
2-(5,6-Dimethoxy-IH-benzimidazol-2-yl sulphinyl methyl)-N,N,3-trimethyl benzenamine
2-(5,6-Diethoxy-IH-benzimidazol-2-yl thio methyl)-4-methoxy-N,N,3,5-tetramethyl benzenamine
2-(5,6-Diethoxy-IH-benzimidazol-2-yl sulphinyl methyl)-4-methoxy-N,N,3,5-tetramethyl benzenamine
2-(4,7-Dimethoxy-IH-benzimidazol-2-yl thio methyl)-N-cyclohexyl-N-methyl benzenamine
2-(4,7-Dimethoxy-IH-benzimidazol-2-yl sulphinyl methyl)-N-cyclohexyl-N-methyl benzenamine
2-(5-Methoxy-IH-benzimidazol-2-yl thio methyl)-N,N-diethyl benzenamine
2-(5-Methoxy-IH-benzimidazol-2-yl sulphinyl methyl)-N,N-diethyl benzenamine
2-(5-Methoxy-IH-benzimidazol-2-yl thiomethyl)-N-cyclohexyl-N-methyl benzenamine

2-(5-Methoxy-IH-benzimidazol-2-yl sulphinylmethyl)-N-cyclohexyl-N-methyl benzenamine
2-(4-Methoxy-IH-benzimidazol-2-yl thio methyl)-N,N-dimethyl benzenamine
2-(4-Methoxy-IH-benzimidazol-2-yl sulphinyl methyl)-N,N-dimethyl benzenamine
2-(4,7-Diethoxy-IH-benzimidazol-2-yl thio methyl)-N,N-dimethyl benzenamine
2-(4,7-Diethoxy-IH-benzimidazol-2-yl sulphinyl methyl)-N,N-dimethyl benzenamine
2-(5,6-Diethoxy-2-IH-benzimidazolylthiomethyl)-N,N-diethyl-3-methyl-benzenamine
2-(5,6-Diethoxy-2-IH-benzimidazolylsulphinylmethyl)-N,N-diethyl-3-methyl-benzenamine
2-(5,6-Diethoxy-2-IH-benzimidazolylthiomethyl)-N,N,5-trimethyl-benzenamine
2-(5,6-Diethoxy-2-IH-benzimidazolylsulphinylmethyl)-N,N,5-trimethyl-benzenamine
2-(5-Methoxy-6-propyloxy-IH-benzimidazol-2-yl thiomethyl)-N,N-dimethyl benzenamine
2-(5-Methoxy-6-propyloxy-IH-benzimidazol-2-yl sulphinylmethyl)-N,N-dimethyl benzenamine
2-(5,6-Diethoxy-IH-benzimidazol-2-yl)thiomethyl)-N,N-diethyl-5-methyl-benzenamine
2-(5,6-Diethoxy-IH-benzimidazol-2-yl sulphinylmethyl)-N,N-diethyl-5-methyl-benzenamine
5,6-Diethoxy-2-[2-methyl-6-(l-piperidyl)phenylmethylthio]-IH-benzimidazole
5,6-Diethoxy-2-[2-methyl-6-(l-piperidyl)phenylmethylsulphinyl]-IH-benzimidazole
2-(5,6-Diethoxy-IH-2-benzimidazolylthiomethyl)-N,N,3,6-tetramethyl-benzenamine
2-(5,6-Diethoxy-IH-2-benzimidazolylsulphinylmethyl)-N,N,3,6-tetramethyl-benzenamine
2-(5-Methoxy-IH-benzimidazol-2-yl thiomethyl)-4-methoxy-N,N,6-trimethyl benzenamine
2-(5-Methoxy-IH-benzimidazol-2-yl sulphinylmethyl)-4-methoxy-N,N,6-trimethyl benzenamine
2-(5-Chloro-IH-benzimidazol-2-yl thiomethyl)-N,N-diethyl benzenamine
2-(5-Chloro-IH-benzimidazol-2-yl sulphinylmethyl)-N,N-diethyl benzenamine
2 (5-Hydroxy-IH-benzimidazol-2-yl thiomethyl)-N,N-dimethyl benzenamine
2-(5-Hydroxy-IH-benzimidazol-2-yl sulphinylmethyl)-N,N-dimethyl benzenamine
N,N-Dimethyl-2-(5-(4-morpholinyl, 4-oxide)-ethoxy]-IH-benzimidazol-2-yl thiomethyl)benzenamine
N,N-Dimethyl-2-(5-(4-morpholinyl, 4-oxide)-ethoxy]-IH-benzimidazol-2-yl sulphinylmethyl)benzenamine
N,N-Dimethyl-2-(5-hydroxy-6-methoxy-IH-benzimidazol-2-yl thiomethyl)benzenamine
N,N-Dimethyl-2-(5-hydroxy-6-methoxy-IH-benzimidazol-2-yl sulphinylmethyl)benzenamine
2-(5,6-Dihydroxy-IH-benzimidazol-2-yl thio methyl)-N,N-dimethyl benzenamine
2-(5,6-Dihydroxy-IH-benzimidazol-2-yl sulphinyl methyl)-N,N-dimethyl benzenamine
2-(5-Hydroxymethyl-IH-benzimidazol-2-yl thio methyl)-N,N-dimethyl benzenamine
2-(5-Hydroxymethyl-IH-benzimidazol-2-yl sulphinyl methyl)-N,N-dimethyl benzenamine
2-(5-Hydroxymethyl-6-methoxy-IH-benzimidazol-2-yl thiomethyl)-N,N-dimethyl benzenamine
2-(5-Hydroxymethyl-6-methoxy-IH-benzimidazol-2-yl sulphinylmethyl)-N,N-dimethyl benzenamine
2-(6,7-Dihydrodioxino[2,3-f]-IH-benzimidazol-2-yl thiomethyl)-N,N-dimethyl benzenamine
2-(6,7-Dihydrodioxino[2,3-f]-IH-benzimidazol-2-yl sulphinylmethyl)-N,N-dimethyl benzenamine
2-(7,8-Dihydrodioxino[2,3-e]-IH-benzimidazol-2-yl thiomethyl)-N,N-dimethyl benzenamine
2-(7,8-Dihydrodioxino[2,3-e]-IH-benzimidazol-2-yl sulphinylmethyl)-N,N-dimethyl benzenamine
2-(2,3-Dihydro-2-oxo-5H-imadazo[4,5-f]benzoxazol-6-yl thiomethyl)-N,N-dimethyl benzenamine
2-(2,3-Dihydro-2-oxo-5H-imadazo[4,5-f]benzoxazol-6-yl sulphinylmethyl)-N,N-dimethyl benzenamine
2-(4,7-Dimethoxy-l-methyl-IH-benzimidazol-2-yl sulphinylmethyl)-N,N-dimethyl benzenamine
2-(5,6-Dimethoxy-l-methyl-IH-benzimidazol-2-yl sulphinylmethyl)-N,N-dimethyl benzenamine
2-(5,6-Dimethoxy-l-propyl-IH-benzimidazol-2-yl sulphinylmethyl)-N,N-dimethyl benzenamine
2-(5,6-Dimethoxy-l-phenylmethyl-IH-benzimidazol-2-yl sulphinylmethyl)-N,N-dimethyl benzenamine
2-(5-Methoxy-l-methyl-IH-benzimidazol-2-yl sulphinyl methyl)-N,N-dimethyl benzenamine
2-(5,6-Diethoxy-l-methyl-IH-benzimidazol-2-yl sulphinyl methyl)-N,N-diethyl benzenamine
N,N,-Diethyl-2-(5,6-dimethoxy-l-methyl-IH-benzimidazol-2-yl sulphinylmethyl)benzenamine
2-(5,6-Dimethoxy-l-phenyl-IH-benzimidazol-2-yl sulphinylmethyl)-N,N,-dimethyl benzenamine
l-Methyl-2-[2-(l-piperidyl)phenylmethylsulphinyl]-IH-benzimidazole
2-(l-Methyl-IH-benzimidazol-2-yl sulphinylmethyl)-N-ethyl-N-propyl benzenamine
2-(l-Prop-2-enyl-IH-benzimidazol-2-yl sulphinyl methyl)-N,N-dimethyl benzenamine
2-(l-Methyl-IH-benzimidazol-2-yl sulphinylmethyl)-4-fluoro-N,N-dimethyl benzenamine
N,N,6-Trimethyl-2-(l-methyl-IH-benzimidazol-2-yl sulphinylmethyl)-benzenamine
2-(l-Methyl-6,7-dihydrodioxino[2,3-f]-IH-benzimidazol-2-yl sulphinylmethyl)-N,N-dimethyl benzenamine,
or
a pharmaceutically acceptable salt of any one thereof.

6. A pharmaceutical formulation comprising a compound according to any one of the preceding claims in admixture with a pharmaceutically acceptable adjuvant, diluent or carrier.

7. The pharmaceutical use of a compound of formula I as defined in Claim I, or a pharmaceutically acceptable salt thereof.

8. The use of a compound of formula I as defined in Claim I to make a pharmaceutical formulation for use as a cytoprotective agent, in the treatment or prophylaxis of inflammatory conditions, or in the prevention or inhibition of gastric acid secretion.

9. A process for the production of a compound of formula I in which n is I by selective oxidation of a compound of formula I in which n is 0,

b) production of a compound of formula I in which $R_{11}$ is not hydrogen, by reaction of a corresponding compound of formula I in which $R_{11}$ is hydrogen with a compound $R_{11}Z$ in which $R_{11}$ is as defined above save that it can not be hydrogen, and Z is a good leaving group,

c) production of a compound of formula I carrying a $-NH_2$ group by selective reduction of a corresponding compound of formula I carrying a $-NO_2$ group, or

d) production of a compound of formula I in which n is 0, by reaction of a compound of formula II,

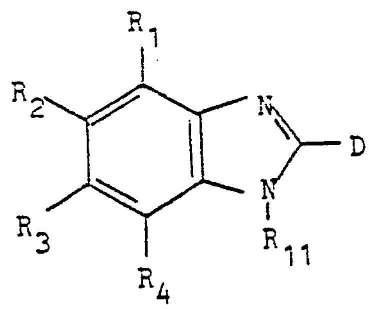

II

in which $R_1$, $R_2$, $R_3$, $R_4$ and $R_{11}$ are as defined above,

with a compound of formula III,

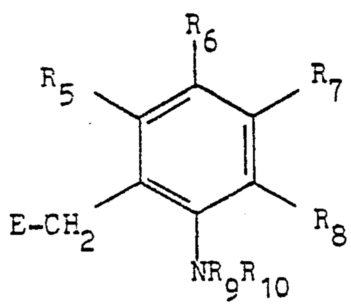

III

in which $R_5$, $R_6$, $R_7$, $R_8$, $R_9$ and $R_{10}$ are as defined above, and

one of D and E is -SH and the other is a good leaving group, eg halogen (chlorine or bromine),

and where desired or necessary converting the resulting compound of formula I to a pharmaceutically acceptable salt thereof, or vice versa.

Claims for the following Contracting States : AT, ES:

I. A process for the production of a compound of formula I,

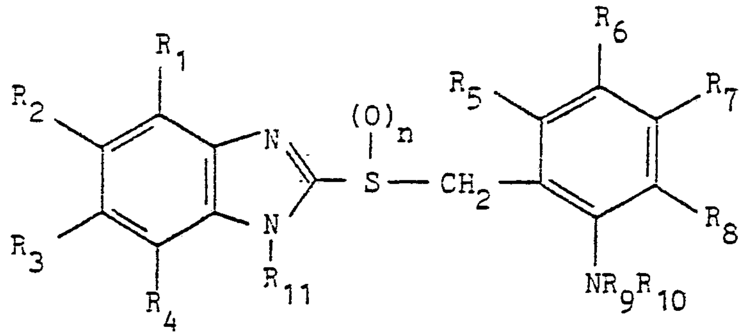

I

in which $R_1$, $R_2$, $R_3$, $R_4$, $R_5$, $R_6$, $R_7$ and $R_8$, which may be the same or different, are each hydrogen, halogen, alkyl C I to C6, $-(CH_2)_mOH$, $-NO_2$, $-NR_{13}R_{14}$, -COOH or an ester thereof, or alkoxy C I to C6 optionally substituted by a saturated heterocyclic ring,

or an adjacent pair of $R_1$, $R_2$, $R_3$ and $R_4$ may, in addition to the values given above, form an $-OCH_2CH_2O$-or -OCONH-chain,

m is 0 or I,

n is 0 or I,

$R_{13}$ and $R_{14}$, which may be the same or different, are each hydrogen or alkyl C I to C6,

$R_{11}$ is hydrogen, phenyl, alkenyl C2 to C6, or alkyl C I to C6 optionally substituted by phenyl,

$R_9$ and $R_{10}$, which may be the same or different, are each cycloalkyl C3 to C7 or alkyl C I to C6, optionally substituted by phenyl, or

$R_9$ and $R_{10}$, together with the nitrogen atom to which they are attached, may form a saturated 6 to 8 inclusive membered ring which contains no heteroatoms other than the nitrogen atom to which $R_9$ and $R_{10}$ are attached,

provided that

a) when $R_{11}$ is hydrogen, then at least one of $R_1$, $R_2$, $R_3$, $R_4$ is other than hydrogen;

b) when $R_5$, $R_6$, $R_{11}$ are each hydrogen and $R_9$, $R_{10}$ are both methyl, then

i) when $R_1$, $R_3$, $R_4$, $R_7$, $R_8$ are each hydrogen, $R_2$ is not chloro, methoxycarbonyl, methyl, methoxy, $-NO_2$ or $-NH_2$;

ii) when $R_1$, $R_4$, $R_7$, $R_8$ are each hydrogen, $R_2$ and $R_3$ do not both represent methyl, chloro or methoxy;

iii) when $R_2$, $R_3$, $R_7$, $R_8$ are each hydrogen, $R_1$ and $R_4$ do not both represent methoxy;

iv) when $R_2$, $R_3$, $R_4$, $R_7$, $R_8$ are each hydrogen, $R_1$ is not methyl;

v) when $R_1$, $R_3$, $R_4$, $R_7$ are each hydrogen and $R_8$ is methyl, $R_2$ is not methoxy;

vi) when $R_1$, $R_3$, $R_4$, $R_8$ are each hygrogen and $R_7$ is methoxy, $R_2$ is not chloro;

c) when $R_1$ to $R_8$ each represent hydrogen, $R_9$, $R_{10}$, $R_{11}$ do not each represent methyl;

d) when $R_1$, $R_4$, $R_7$, $R_3$ and $R_{11}$ are each hydrogen, $R_9$ and $R_{10}$ are both ethyl, $R_2$ and $R_3$ are both methoxy and one of $R_5$ or $R_6$ is hydrogen the remainder of $R_5$ or $R_6$ is not hydrogen or methyl,

and pharmaceutically acceptable salts thereof, which comprises

a) production of a compound of formula I in which n is I by selective oxidation of a compound of formula I in which n is 0,

b) production of a compound of formula I in which $R_{11}$ is not hydrogen, by reaction of a corresponding compound of formula I in which $R_{11}$ is hydrogen with a compound $R_{11}Z$ in which $R_{11}$ is as defined above save that it can not be hydrogen, and Z is a good leaving group,

c) production of a compound of formula I carrying a $-NH_2$ group by selective reduction of a corresponding compound of formula I carrying a $-NO_2$ group, or

d) production of a compound of formula I in which n is 0, by reaction of a compound of formula II,

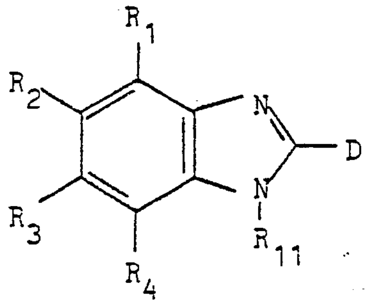

II

in which $R_1$, $R_2$, $R_3$, $R_4$ and $R_{11}$ are as defined above, with a compound of formula III,

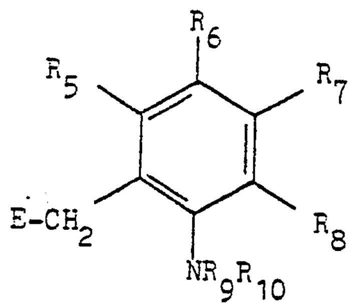

III

in which $R_5$, $R_6$, $R_7$, $R_8$, $R_9$ and $R_{10}$ are as defined above, and

one of D and E is -SH and the other is a good leaving group, eg halogen (chlorine or bromine),

and where desired or necessary converting the resulting compound of formula I to a pharmaceutically acceptable salt thereof, or vice versa.

2. A process according to Claim I, in which when any of $R_1$ to $R_8$ is halogen it is fluorine or chlorine,

when any of $R_1$ to $R_8$ represent an ester it is a methyl or ethyl ester,

when any of $R_1$ to $R_8$ represent alkoxy substituted by a saturated heterocyclic ring, that ring is a morpholino ring, and

when $R_9$ and $R_{10}$ together with the nitrogen atom to which they are attached, form a ring, that ring is a piperidino ring.

3. A process according to Claim I or Claim 2, in which $R_1$ to $R_8$ are each selected from hydrogen, methoxycarbonyl, methyl, butyl, chloro, fluoro, methoxy, ethoxy, propyloxy, butyloxy, hydroxy, hydroxymethyl, $-NO_2$ or alkoxy substituted by morpholine-N-oxide, or

in addition to the values above $R_5$ and $R_6$ or $R_6$ and $R_7$ form an $-OCH_2CH_2O-$or -OCONH-chain,

$R_{11}$ is hydrogen, methyl, propyl, benzyl, phenyl or prop-2-enyl, and

$R_9$ and $R_{10}$, which may be the same or different, are each methyl, ethyl, propyl or cyclohexyl, or

$R_9$ and $R_{10}$ together with the nitrogen atom to which they are attached, form a pyridine ring.

4. A process according to any of the above claims, in which at least two of $R_1$ to $R_4$ is alkoxy,

$R_{11}$ is hydrogen or alkyl C I to C6,

n is I, and

$R_9$ and $R_{10}$ are both alkyl or together comprise more than 3 carbon atoms.

5. A process according to Claim I, in which at least two of $R_1$ to $R_4$ are methoxy or ethoxy,

$R_{11}$ is hydrogen or methyl,

n is I, and

$R_9$ and $R_{10}$ are methyl, ethyl or together comprise more than 3 carbon atoms.

6. A process according to Claim I, in which $R_2$ and $R_3$ are both alkoxy,

n is I, and

$R_9$ and $R_{10}$ are both methyl or both ethyl.

7. A process according to Claim I, in which $R_2$ and $R_3$ are both methoxy or both ethoxy,

n is I, and

$R_9$ and $R_{10}$ are both ethyl.

8. A process according to Claim I, wherein the compound of formula is,
2-(5,6-Diethoxy-IH-benzimidazol-2-yl sulphinylmethyl)-N,N-diethyl benzenamine.
9. A process according to Claim I, when the compound of formula I is,
2-(5,6-Diethoxy-IH-benzimidazol-2-yl thiomethyl)-N,N-diethyl benzenamine
2-(4,5-Dimethoxy-IH-benzimidazol-2-yl thio methyl)-N,N-dimethyl benzenamine
2-(4,5-Dimethoxy-IH-benzimidazol-2-yl sulphinyl methyl)-N,N-dimethyl benzenamine
2-(4,7-Dimethoxy-IH-benzimidazol-2-yl thio methyl)-N,N-4-trimethyl benzenamine
2-(4,7-Dimethoxy-IH-benzimidazol-2-yl sulphinyl methyl)-N,N-4-trimethyl benzenamine
2-(4,5,6-Trimethoxy-IH-benzimidazol-2-yl thio methyl)-N,N-dimethyl benzenamine
2-(4,5,6-Trimethoxy-IH-benzimidazol-2-yl sulphinyl methyl)-N,N-dimethyl benzenamine
2-(4,6-Dimethoxy-IH-benzimidazol-2-yl thio methyl)-N,N-dimethyl benzenamine
2-(4,6-Dimethoxy-IH-benzimidazol-2-yl sulphinyl methyl)-N,N-dimethyl benzenamine
Methyl 6-methoxy-2-(2-dimethylamino-phenylmethyl thio)-IH-benzimidazol-5-carboxylate
Methyl 6-methoxy-2-(2-dimethylamino-phenylmethyl sulphinyl)-IH-benzimidazol-5-carboxylate
2-(5-Methoxy-IH-benzimidazol-2-yl thio methyl)-N,N-dimethyl-4-(I,I-dimethylethyl)benzenamine
2-(5-Methoxy-IH-benzimidazol-2-yl sulphinyl methyl)-N,N-dimethyl-4-(I,I-dimethylethyl)benzenamine
2-(5,6-Dimethoxy-IH-benzimidazol-2-yl thio methyl)-5-nitro-N,N-dimethyl benzenamine
2-(5,6-Dimethoxy-IH-benzimidazol-2-yl sulphinyl methyl)-5-nitro-N,N-dimethyl benzenamine
2-(5,6-Dimethoxy-IH-benzimidazol-2-yl thio methyl)-N,N,4-trimethyl benzenamine
2-(5,6-Dimethoxy-IH-benzimidazol-2-yl sulphinyl methyl)-N,N,4-trimethyl benzenamine
2-(5,6-Dimethoxy-IH-benzimidazol-2-yl thio methyl)-N,N-dimethyl-4-(I,I-dimethylethyl)benzenamine
2-(5,6-Dimethoxy-IH-benzimidazol-2-yl sulphinyl methyl)-N,N-dimethyl-4-(I,I-dimethylethyl)benzenamine
2-(5,6-Diethoxy-IH-benzimidazol-2-yl thio methyl)-N,N-dimethyl benzenamine
2-(5,6-Diethoxy-IH-benzimidazol-2-yl sulphinyl methyl)-N,N-dimethyl benzenamine
2-(5,6-Diethoxy-IH-benzimidazol-2-yl thio methyl)-N-ethyl-N-methyl benzenamine
2-(5,6-Diethoxy-IH-benzimidazol-2-yl sulphinyl methyl)-N-ethyl-N-methyl benzenamine
2-(5-Butyloxy-6-ethoxy-IH-benzimidazol-2-yl thio methyl)-N,N-dimethyl benzenamine
2-(5-Butyloxy-6-ethoxy-IH-benzimidazol-2-yl sulphinyl methyl)-N,N-dimethyl benzenamine
2-(4,5,7-Trimethoxy-IH-benzimidazol-2-yl thio methyl)-N,N-dimethyl benzenamine
2-(4,5,7-Trimethoxy-IH-benzimidazol-2-yl sulphinyl methyl)-N,N-dimethyl benzenamine
2-(5,6-Diethoxy-IH-benzimidazol-2-yl thio methyl)-N,N,4-trimethyl benzenamine
2-(5,6-Diethoxy-IH-benzimidazol-2-yl sulphinyl methyl)-N,N,4-trimethyl benzenamine
2-(5-Methoxy-IH-benzimidazol-2-yl thio methyl)-N,N,3-trimethyl benzenamine
2-(5-Methoxy-IH-benzimidazol-2-yl sulphinyl methyl)-N,N,3-trimethyl benzenamine
2-(5,6-Diethoxy-IH-benzimidazol-2-yl thio methyl)-N,N,3-trimethyl benzenamine
2-(5,6-Diethoxy-IH-benzimidazol-2-yl sulphinyl methyl)-N,N,3-trimethyl benzenamine
2-(5,6-Dimethoxy-IH-benzimidazol-2-yl thio methyl)-N,N,3-trimethyl benzenamine
2-(5,6-Dimethoxy-IH-benzimidazol-2-yl sulphinyl methyl)-N,N,3-trimethyl benzenamine
2-(5,6-Diethoxy-IH-benzimidazol-2-yl thio methyl)-4-methoxy-N,N,3,5-tetramethyl benzenamine
2-(5,6-Diethoxy-IH-benzimidazol-2-yl sulphinyl methyl)-4-methoxy-N,N,3,5-tetramethyl benzenamine
2-(4,7-Dimethoxy-IH-benzimidazol-2-yl thio methyl)-N-cyclohexyl-N-methyl benzenamine
2-(4,7-Dimethoxy-IH-benzimidazol-2-yl sulphinyl methyl)-N-cyclohexyl-N-methyl benzenamine
2-(5-Methoxy-IH-benzimidazol-2-yl thio methyl)-N,N-diethyl benzenamine
2-(5-Methoxy-IH-benzimidazol-2-yl sulphinyl methyl)-N,N-diethyl benzenamine
2-(5-Methoxy-IH-benzimidazol-2-yl thiomethyl)-N-cyclohexyl-N-methyl benzenamine
2-(5-Methoxy-IH-benzimidazol-2-yl sulphinylmethyl)-N-cyclohexyl-N-methyl benzenamine
2-(4-Methoxy-IH-benzimidazol-2-yl thio methyl)-N,N-dimethyl benzenamine
2-(4-Methoxy-IH-benzimidazol-2-yl sulphinyl methyl)-N,N-dimethyl benzenamine
2-(4,7-Diethoxy-IH-benzimidazol-2-yl thio methyl)-N,N-dimethyl benzenamine
2-(4,7-Diethoxy-IH-benzimidazol-2-yl sulphinyl methyl)-N,N-dimethyl benzenamine
2-(5,6-Diethoxy-2-IH-benzimidazolylthiomethyl)-N,N-diethyl-3-methyl-benzenamine
2-(5,6-Diethoxy-2-IH-benzimidazolylsulphinylmethyl)-N,N-diethyl-3-methyl-benzenamine
2-(5,6-Diethoxy-2-IH-benzimidazolylthiomethyl)-N,N,5-trimethyl-benzenamine
2-(5,6-Diethoxy-2-IH-benzimidazolylsulphinylmethyl)-N,N,5-trimethyl-benzenamine
2-(5-Methoxy-6-propyloxy-IH-benzimidazol-2-yl thiomethyl)-N,N-dimethyl benzenamine
2-(5-Methoxy-6-propyloxy-IH-benzimidazol-2-yl sulphinylmethyl)-N,N-dimethyl benzenamine
2-(5,6-Diethoxy-IH-benzimidazol-2-yl)thiomethyl)-N,N-diethyl-5-methyl-benzenamine
2-(5,6-Diethoxy-IH-benzimidazol-2-yl sulphinylmethyl)-N,N-diethyl-5-methyl-benzenamine

5,6-Diethoxy-2-[2-methyl-6-(l-piperidyl)phenylmethyl thio]-IH-benzimidazole
5,6-Diethoxy-2-[2-methyl-6-(l-piperidyl)phenylmethylsulphinyl]-IH-benzimidazole
2-(5,6-Diethoxy-IH-2-benzimidazolylthiomethyl)-N,N,3,6-tetramethyl-benzenamine
2-(5,6-Diethoxy-IH-2-benzimidazolylsulphinylmethyl)-N,N,3,6-tetramethyl-benzenamine
2-(5-Methoxy-IH-benzimidazol-2-yl thiomethyl)-4-methoxy-N,N,6-trimethyl benzenamine
2-(5-Methoxy-IH-benzimidazol-2-yl sulphinylmethyl)-4-methoxy-N,N,6-trimethyl benzenamine
2-(5-Chloro-IH-benzimidazol-2-yl thiomethyl)-N,N-diethyl benzenamine
2-(5-Chloro-IH-benzimidazol-2-yl sulphinylmethyl)-N,N-diethyl benzenamine
2-(5-Hydroxy-IH-benzimidazol-2-yl thiomethyl)-N,N-dimethyl benzenamine
2-(5-Hydroxy-IH-benzimidazol-2-yl sulphinylmethyl)-N,N-dimethyl benzenamine
N,N-Dimethyl-2-(5-(4-morpholinyl, 4-oxide)-ethoxy]-IH-benzimidazol-2-yl thiomethyl)benzenamine
N,N-Dimethyl-2-(5-(4-morpholinyl, 4-oxide)-ethoxy]-IH-benzimidazol-2-yl sulphinylmethyl)benzenamine
N,N-Dimethyl-2-(5-hydroxy-6-methoxy-IH-benzimidazol-2-yl thiomethyl)benzenamine
N,N-Dimethyl-2-(5-hydroxy-6-methoxy-IH-benzimidazol-2-yl sulphinylmethyl)benzenamine
2-(5,6-Dihydroxy-IH-benzimidazol-2-yl thio methyl)-N,N-dimethyl benzenamine
2-(5,6-Dihydroxy-IH-benzimidazol-2-yl sulphinyl methyl)-N,N-dimethyl benzenamine
2-(5-Hydroxymethyl-IH-benzimidazol-2-yl thio methyl)-N,N-dimethyl benzenamine
2-(5-Hydroxymethyl-IH-benzimidazol-2-yl sulphinyl methyl)-N,N-dimethyl benzenamine
2-(5-Hydroxymethyl-6-methoxy-IH-benzimidazol-2-yl thiomethyl)-N,N-dimethyl benzenamine
2-(5-Hydroxymethyl-6-methoxy-IH-benzimidazol-2-yl sulphinylmethyl)-N,N-dimethyl benzenamine
2-(6,7-Dihydrodioxino[2,3-f]-IH-benzimidazol-2-yl thiomethyl)-N,N-dimethyl benzenamine
2-(6,7-Dihydrodioxino[2,3-f]-IH-benzimidazol-2-yl sulphinylmethyl)-N,N-dimethyl benzenamine
2-(7,8-Dihydrodioxino[2,3-e]-IH-benzmidazol-2-yl thiomethyl)-N,N-dimethyl benzenamine
2-(7,8-Dihydrodioxino[2,3-e]-IH-benzimidazol-2-yl sulphinylmethyl)-N,N-dimethyl benzenamine
2-(2,3-Dihydro-2-oxo-5H-imadazo[4,5-f]benzoxazol-6-yl thiomethyl)-N,N-dimethyl benzenamine
2-(2,3-Dihydro-2-oxo-5H-imadazo[4,5-f]benzoxazol-6-yl sulphinylmethyl)-N,N-dimethyl benzenamine
2-(4,7-Dimethoxy-l-methyl-IH-benzimidazol-2-yl sulphinylmethyl)-N,N-dimethyl benzenamine
2-(5,6-Dimethoxy-l-methyl-IH-benzimidazol-2-yl sulphinylmethyl)-N,N-dimethyl benzenamine
2-(5,6-Dimethoxy-l-propyl-IH-benzimidazol-2-yl sulphinylmethyl)-N,N-dimethyl benzenamine
2-(5,6-Dimethoxy-l-phenylmethyl-IH-benzimidazol-2-yl sulphinylmethyl)-N,N-dimethyl benzenamine
2-(5-Methoxy-l-methyl-IH-benzimidazol-2-yl sulphinyl methyl)-N,N-dimethyl benzenamine
2-(5,6-Diethoxy-l-methyl-IH-benzimidazol-2-yl sulphinyl methyl)-N,N-diethyl benzenamine
N,N,-Diethyl-2-(5,6-dimethoxy-l-methyl-IH-benzimidazol-2-yl sulphinylmethyl)benzenamine
2-(5,6-Dimethoxy-l-phenyl-IH-benzimidazol-2-yl sulphinylmethyl)-N,N,-dimethyl benzenamine
l-Methyl-2-[2-(l-piperidyl)phenylmethylsulphinyl]-IH-benzimidazole
2-(l-Methyl-IH-benzimidazol-2-yl sulphinylmethyl)-N-ethyl-N-propyl benzenamine
2-(l-Prop-2-enyl-IH-benzimidazol-2-yl sulphinyl methyl)-N,N-dimethyl benzenamine
2-(l-Methyl-IH-benzimidazol-2-yl sulphinylmethyl)-4-fluoro-N,N-dimethyl benzenamine
N,N,6-Trimethyl-2-(l-methyl-IH-benzimidazol-2-yl sulphinylmethyl)-benzenamine
2-(l-Methyl-6,7-dihydrodioxino[2,3-f]-IH-benzimidazol-2-yl sulphinylmethyl)-N,N-dimethyl benzenamine,

or

a pharmaceutically acceptable salt of any one thereof.

Claims for the following Contracting State : GR

I. A process for the production of a compound of formula I,

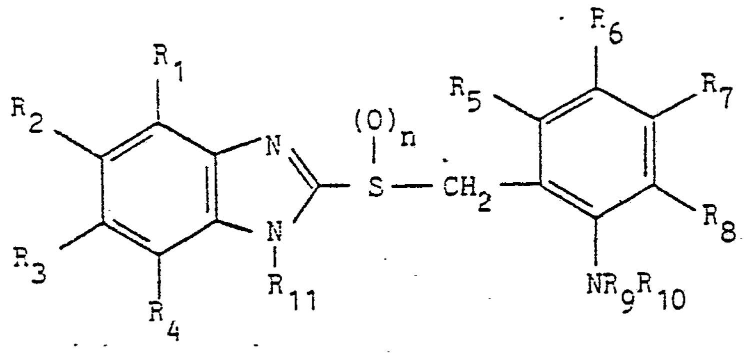

I

in which $R_1$, $R_2$, $R_3$, $R_4$, $R_5$, $R_6$, $R_7$ and $R_8$, which may be the same or different, are each hydrogen, halogen, alkyl C I to C6, $-(CH_2)_mOH$, $-NO_2$, $-NR_{13}R_{14}$, -COOH or an ester thereof, or alkoxy C I to C6 optionally substituted by a saturated heterocyclic ring,

or an adjacent pair of $R_1$, $R_2$, $R_3$ and $R_4$ may, in addition to the values given above, form an $-OCH_2CH_2O-$or -OCONH-chain,

m is 0 or I,

n is 0 or I,

$R_{13}$ and $R_{14}$, which may be the same or different, are each hydrogen or alkyl C I to C6,

$R_{11}$ is hydrogen, phenyl, alkenyl C2 to C6, or alkyl C I to C6 optionally substituted by phenyl,

$R_9$ and $R_{10}$, which may be the same or different, are each cycloalkyl C3 to C7 or alkyl C I to C6, optionally substituted by phenyl, or

$R_9$ and $R_{10}$, together with the nitrogen atom to which they are attached, may form a saturated 6 to 8 inclusive membered ring which contains no heteroatoms other than the nitrogen atom to which $R_9$ and $R_{10}$ are attached,

provided that

a) when $R_{11}$ is hydrogen, then at least one of $R_1$, $R_2$, $R_3$, $R_4$ is other than hydrogen;

b) when $R_5$, $R_6$, $R_{11}$ are each hydrogen and $R_9$, $R_{10}$ are both methyl, then

i) when $R_1$, $R_3$, $R_4$, $R_7$, $R_8$ are each hydrogen, $R_2$ is not chloro, methoxycarbonyl, methyl, methoxy, $-NO_2$ or $-NH_2$;

ii) when $R_1$, $R_4$, $R_7$, $R_8$ are each hydrogen, $R_2$ and $R_3$ do not both represent methyl, chloro or methoxy;

iii) when $R_2$, $R_3$, $R_7$, $R_8$ are each hydrogen, $R_1$ and $R_4$ do not both represent methoxy;

iv) when $R_2$, $R_3$, $R_4$, $R_7$, $R_8$ are each hydrogen, $R_1$ is not methyl;

v) when $R_1$, $R_3$, $R_4$, $R_7$ are each hydrogen and $R_8$ is methyl, $R_2$ is not methoxy ;

vi) when $R_1$, $R_3$, $R_4$, $R_8$ are each hydrogen and $R_7$ is methoxy, $R_2$ is not chloro;

c) when $R_1$ to $R_8$ each represent hydrogen, $R_9$, $R_{10}$, $R_{11}$ do not each represent methyl;

d) when $R_1$, $R_4$, $R_7$, $R_8$ and $R_{11}$ are each hydrogen, $R_9$ and $R_{10}$ are both ethyl, $R_2$ and $R_3$ are both methoxy and one of $R_5$ or $R_6$ is hydrogen the remainder of $R_5$ or $R_6$ is not hydrogen or methyl,

and pharmaceutically acceptable salts thereof, which comprises

a) production of a compound of formula I in which n is I by selective oxidation of a compound of formula I

in which n is 0,

b) production of a compound of formula I in which $R_{11}$ is not hydrogen, by reaction of a corresponding compound of formula I in which $R_{11}$ is hydrogen with a compound $R_{11}Z$ in which $R_{11}$ is as defined above save that it can not be hydrogen, and Z is a good leaving group,

c) production of a compound of formula I carrying a $-NH_2$ group by selective reduction of a corresponding compound of formula I carrying a $-NO_2$ group, or

d) production of a compound of formula I in which n is 0, by reaction of a compound of formula II,

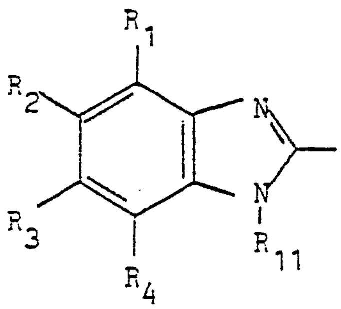

II

in which $R_1$, $R_2$, $R_3$, $R_4$ and $R_{11}$ are as defined above,
with a compound of formula I,

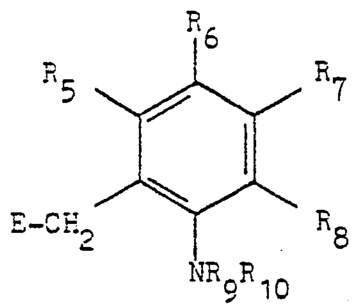

III

in which $R_5$, $R_6$, $R_7$, $R_8$, $R_9$ and $R_{10}$ are as defined above, and
one of D and E is -SH and the other is a good leaving group, eg halogen (chlorine or bromine),
and where desired or necessary converting the resulting compound of formula I to a pharmaceutically acceptable salt thereof, or vice versa.

2. A process according to Claim I, in which when any of $R_1$ to $R_8$ is halogen it is fluorine or chlorine,
when any of $R_1$ to $R_8$ represent an ester it is a methyl or ethyl ester,
when any of $R_1$ to $R_8$ represent alkoxy substituted by a saturated heterocyclic ring, that ring is a morpholino ring, and
when $R_9$ and $R_{10}$ together with the nitrogen atom to which they are attached, form a ring, that ring is a piperidino ring.

3. A process according to Claim I or Claim 2, in which $R_1$ to $R_8$ are each selected from hydrogen, methoxycarbonyl, methyl, butyl, chloro, fluoro, methoxy, ethoxy, propyloxy, butyloxy, hydroxy, hydroxymethyl, $-NO_2$ or alkoxy substituted by morpholine-N-oxide, or
in addition to the values above $R_5$ and $R_6$ or $R_6$ and $R_7$ form an $-OCH_2CH_2O-$or -OCONH-chain,
$R_{11}$ is hydrogen, methyl, propyl, benzyl, phenyl or prop-2-enyl, and
$R_9$ and $R_{10}$, which may be the same or different, are each methyl, ethyl, propyl or cyclohexyl, or
$R_9$ and $R_{10}$ together with the nitrogen atom to which they are attached, form a pyridine ring.

4. A process according to any of the above claims, in which at least two of $R_1$ to $R_4$ is alkoxy,
$R_{11}$ is hydrogen or alkyl C I to C6,
n is I, and
$R_9$ and $R_{10}$ are both alkyl or together comprise more than 3 carbon atoms.

5. A process according to Claim I, in which at least two of $R_1$ to $R_4$ are methoxy or ethoxy,
$R_{11}$ is hydrogen or methyl,
n is I, and
$R_9$ and $R_{10}$ are methyl, ethyl or together comprise more than 3 carbon atoms.

6. A process according to any of the above claims, in which $R_2$ and $R_3$ are both alkoxy,
n is I, and
$R_9$ and $R_{10}$ are both methyl or both ethyl.

7. A process according to any of the above claims, in which $R_2$ and $R_3$ are both methoxy or both ethoxy,
n is I, and
$R_9$ and $R_{10}$ are both ethyl.

8. A process according to Claim I, wherein the compound of formula is,
2-(5,6-Diethoxy-IH-benzimidazol-2-yl sulphinylmethyl)-N,N-diethyl benzenamine.
9. A process according to Claim I, when the compound of formula I is,
2-(5,6-Diethoxy-IH-benzimidazol-2-yl thiomethyl)-N,N-diethyl benzenamine
2-(4,5-Dimethoxy-IH-benzimidazol-2-yl thio methyl)-N,N-dimethyl benzenamine
2-(4,5-Dimethoxy-IH-benzimidazol-2-yl sulphinyl methyl)-N,N-dimethyl benzenamine
2-(4,7-Dimethoxy-IH-benzimidazol-2-yl thio methyl)-N,N-4-trimethyl benzenamine
2-(4,7-Dimethoxy-IH-benzimidazol-2-yl sulphinyl methyl)-N,N-4-trimethyl benzenamine
2-(4,5,6-Trimethoxy-IH-benzimidazol-2-yl thio methyl)-N,N-dimethyl benzenamine
2-(4,5,6-Trimethoxy-IH-benzimidazol-2-yl sulphinyl methyl)-N,N-dimethyl benzenamine
2-(4,6-Dimethoxy-IH-benzimidazol-2-yl thio methyl)-N,N-dimethyl benzenamine
2-(4,6-Dimethoxy-IH-benzimidazol-2-yl sulphinyl methyl)-N,N-dimethyl benzenamine
Methyl 6-methoxy-2-(2-dimethylamino-phenylmethyl thio)-IH-benzimidazol-5-carboxylate
Methyl 6-methoxy-2-(2-dimethylamino-phenylmethyl sulphinyl)-IH-benzimidazol-5-carboxylate
2-(5-Methoxy-IH-benzimidazol-2-yl thio methyl)-N,N-dimethyl-4-(I,I-dimethylethyl)benzenamine
2-(5-Methoxy-IH-benzimidazol-2-yl sulphinyl methyl)-N,N-dimethyl-4-(I,I-dimethylethyl)benzenamine
2-(5,6-Dimethoxy-IH-benzimidazol-2-yl thio methyl)-5-nitro-N,N-dimethyl benzenamine
2-(5,6-Dimethoxy-IH-benzimidazol-2-yl sulphinyl methyl)-5-nitro-N,N-dimethyl benzenamine
2-(5,6-Dimethoxy-IH-benzimidazol-2-yl thio methyl)-N,N,4-trimethyl benzenamine
2-(5,6-Dimethoxy-IH-benzimidazol-2-yl sulphinyl methyl)-N,N,4-trimethyl benzenamine
2-(5,6-Dimethoxy-IH-benzimidazol-2-yl thio methyl)-N,N-dimethyl-4-(I,I-dimethylethyl)benzenamine
2-(5,6-Dimethoxy-IH-benzimidazol-2-yl sulphinyl methyl)-N,N-dimethyl-4-(I,I-dimethylethyl)benzenamine
2-(5,6-Diethoxy-IH-benzimidazol-2-yl thio methyl)-N,N-dimethyl benzenamine
2-(5,6-Diethoxy-IH-benzimidazol-2-yl sulphinyl methyl)-N,N-dimethyl benzenamine
2-(5,6-Diethoxy-IH-benzimidazol-2-yl thio methyl)-N-ethyl-N-methyl benzenamine
2-(5,6-Diethoxy-IH-benzimidazol-2-yl sulphinyl methyl)-N-ethyl-N-methyl benzenamine
2-(5-Butyloxy-6-ethoxy-IH-benzimidazol-2-yl thio methyl)-N,N-dimethyl benzenamine
2-(5-Butyloxy-6-ethoxy-IH-benzimidazol-2-yl sulphinyl methyl)-N,N-dimethyl benzenamine
2-(4,5,7-Trimethoxy-IH-benzimidazol-2-yl thio methyl)-N,N-dimethyl benzenamine
2-(4,5,7-Trimethoxy-IH-benzimidazol-2-yl sulphinyl methyl)-N,N-dimethyl benzenamine
2-(5,6-Diethoxy-IH-benzimidazol-2-yl thio methyl)-N,N,4-trimethyl benzenamine
2-(5,6-Diethoxy-IH-benzimidazol-2-yl sulphinyl methyl)-N,N,4-trimethyl benzenamine
2-(5-Methoxy-IH-benzimidazol-2-yl thio methyl)-N,N,3-trimethyl benzenamine
2-(5-Methoxy-IH-benzimidazol-2-yl sulphinyl methyl)-N,N,3-trimethyl benzenamine
2-(5,6-Diethoxy-IH-benzimidazol-2-yl thio methyl)-N,N,3-trimethyl benzenamine
2-(5,6-Diethoxy-IH-benzimidazol-2-yl sulphinyl methyl)-N,N,3-trimethyl benzenamine
2-(5,6-Dimethoxy-IH-benzimidazol-2-yl thio methyl)-N,N,3-trimethyl benzenamine
2-(5,6-Dimethoxy-IH-benzimidazol-2-yl sulphinyl methyl)-N,N,3-trimethyl benzenamine
2-(5,6-Diethoxy-IH-benzimidazol-2-yl thio methyl)-4-methoxy-N,N,3,5-tetramethyl benzenamine
2-(5,6-Diethoxy-IH-benzimidazol-2-yl sulphinyl methyl)-4-methoxy-N,N,3,5-tetramethyl benzenamine
2-(4,7-Dimethoxy-IH-benzimidazol-2-yl thio methyl)-N-cyclohexyl-N-methyl benzenamine
2-(4,7-Dimethoxy-IH-benzimidazol-2-yl sulphinyl methyl)-N-cyclohexyl-N-methyl benzenamine
2-(5-Methoxy-IH-benzimidazol-2-yl thio methyl)-N,N-diethyl benzenamine
2-(5-Methoxy-IH-benzimidazol-2-yl sulphinyl methyl)-N,N-diethyl benzenamine
2-(5-Methoxy-IH-benzimidazol-2-yl thiomethyl)-N-cyclohexyl-N-methyl benzenamine
2-(5-Methoxy-IH-benzimidazol-2-yl sulphinylmethyl)-N-cyclohexyl-N-methyl benzenamine
2-(4-Methoxy-IH-benzimidazol-2-yl thio methyl)-N,N-dimethyl benzenamine
2-(4-Methoxy-IH-benzimidazol-2-yl sulphinyl methyl)-N,N-dimethyl benzenamine
2-(4,7-Diethoxy-IH-benzimidazol-2-yl thio methyl)-N,N-dimethyl benzenamine
2-(4,7-Diethoxy-IH-benzimidazol-2-yl sulphinyl methyl)-N,N-dimethyl benzenamine
2-(5,6-Diethoxy-2-IH-benzimidazolylthiomethyl)-N,N-diethyl-3-methyl-benzenamine
2-(5,6-Diethoxy-2-IH-benzimidazolylsulphinylmethyl)-N,N-diethyl-3-methyl-benzenamine
2-(5,6-Diethoxy-2-IH-benzimidazolylthiomethyl)-N,N,5-trimethyl-benzenamine
2-(5,6-Diethoxy-2-IH-benzimidazolylsulphinylmethyl)-N,N,5-trimethyl-benzenamine
2-(5-Methoxy-6-propyloxy-IH-benzimidazol-2-yl thiomethyl)-N,N-dimethyl benzenamine
2-(5-Methoxy-6-propyloxy-IH-benzimidazol-2-yl sulphinylmethyl)-N,N-dimethyl benzenamine
2-(5,6-Diethoxy-IH-benzimidazol-2-yl)thiomethyl)-N,N-diethyl-5-methyl-benzenamine
2-(5,6-Diethoxy-IH-benzimidazol-2-yl sulphinylmethyl)-N,N-diethyl-5-methyl-benzenamine

5,6-Diethoxy-2-[2-methyl-6-(l-piperidyl)phenylmethyl thio]-lH-benzimidazole
5,6-Diethoxy-2-[2-methyl-6-(l-piperidyl)phenylmethylsulphinyl]-lH-benzimidazole
2-(5,6-Diethoxy-lH-2-benzimidazolylthiomethyl)-N,N,3,6-tetramethyl-benzenamine
2-(5,6-Diethoxy-lH-2-benzimidazolylsulphinylmethyl)-N,N,3,6-tetramethyl-benzenamine
2-(5-Methoxy-lH-benzimidazol-2-yl thiomethyl)-4-methoxy-N,N,6-trimethyl benzenamine
2-(5-Methoxy-lH-benzimidazol-2-yl sulphinylmethyl)-4-methoxy-N,N,6-trimethyl benzenamine
2-(5-Chloro-lH-benzimidazol-2-yl thiomethyl)-N,N-diethyl benzenamine
2-(5-Chloro-lH-benzimidazol-2-yl sulphinylmethyl)-N,N-diethyl benzenamine
2-(5-Hydroxy-lH-benzimidazol-2-yl thiomethyl)-N,N-dimethyl benzenamine
2-(5-Hydroxy-lH-benzimidazol-2-yl sulphinylmethyl)-N,N-dimethyl benzenamine
N,N-Dimethyl-2-(5-(4-morpholinyl, 4-oxide)-ethoxy]-lH-benzimidazol-2-yl thiomethyl)benzenamine
N,N-Dimethyl-2-(5-(4-morpholinyl, 4-oxide)-ethoxy]-lH-benzimidazol-2-yl sulphinylmethyl)benzenamine
N,N-Dimethyl-2-(5-hydroxy-6-methoxy-lH-benzimidazol-2-yl thiomethyl)benzenamine
N,N-Dimethyl-2-(5-hydroxy-6-methoxy-lH-benzimdazol-2-yl sulphinylmethyl)benzenamine
2-(5,6-Dihydroxy-lH-benzimidazol-2-yl thio methyl)-N,N-dimethyl benzenamine
2-(5,6-Dihydroxy-lH-benzimidazol-2-yl sulphinyl methyl)-N,N-dimethyl benzenamine
2-(5-Hydroxymethyl-lH-benzimidazol-2-yl thio methyl)-N,N-dimethyl benzenamine
2-(5-Hydroxymethyl-lH-benzimidazol-2-yl sulphinyl methyl)-N,N-dimethyl benzenamine
2-(5-Hydroxymethyl-6-methoxy-lH-benzimidazol-2-yl thiomethyl)-N,N-dimethyl benzenamine
2-(5-Hydroxymethyl-6-methoxy-lH-benzimidazol-2-yl sulphinylmethyl)-N,N-dimethyl benzenamine
2-(6,7-Dihydrodioxino[2,3-f]-lH-benzimidazol-2-yl thiomethyl)-N,N-dimethyl benzenamine
2-(6,7-Dihydrodioxino[2,3-f]-lH-benzimidazol-2-yl sulphinylmethyl)-N,N-dimethyl benzenamine
2-(7,8-Dihydrodioxino[2,3-e]-lH-benzimidazol-2-yl thiomethyl)-N,N-dimethyl benzenamine
2-(7,8-Dihydrodioxino[2,3-e]-lH-benzimidazol-2-yl sulphinylmethyl)-N,N-dimethyl benzenamine
2-(2,3-Dihydro-2-oxo-5H-imadazo[4,5-f]benzoxazol-6-yl thiomethyl)-N,N-dimethyl benzenamine
2-(2,3-Dihydro-2-oxo-5H-imadazo[4,5-f]benzoxazol-6-yl sulphinylmethyl)-N,N-dimethyl benzenamine
2-(4,7-Dimethoxy-l-methyl-lH-benzimidazol-2-yl sulphinylmethyl)-N,N-dimethyl benzenamine
2-(5,6-Dimethoxy-l-methyl-lH-benzimidazol-2-yl sulphinylmethyl)-N,N-dimethyl benzenamine
2-(5,6-Dimethoxy-l-propyl-lH-benzimidazol-2-yl sulphinylmethyl)-N,N-dimethyl benzenamine
2-(5,6-Dimethoxy-l-phenylmethyl-lH-benzimidazol-2-yl sulphinylmethyl)-N,N-dimethyl benzenamine
2-(5-Methoxy-l-methyl-lH-benzimidazol-2-yl sulphinyl methyl)-N,N-dimethyl benzenamine
2-(5,6-Diethoxy-l-methyl-lH-benzimidazol-2-yl sulphinyl methyl)-N,N-diethyl benzenamine
N,N,-Diethyl-2-(5,6-dimethoxy-l-methyl-lH-benzimidazol-2-yl sulphinylmethyl)benzenamine
2-(5,6-Dimethoxy-l-phenyl-lH-benzimidazol-2-yl sulphinylmethyl)-N,N,-dimethyl benzenamine
l-Methyl-2-[2-(l-piperidyl)phenylmethylsulphinyl]-lH-benzimidazole
2-(l-Methyl-lH-benzimidazol-2-yl sulphinylmethyl)-N-ethyl-N-propyl benzenamine
2-(l-Prop-2-enyl-lH-benzimidazol-2-yl sulphinyl methyl)-N,N-dimethyl benzenamine
2-(l-Methyl-lH-benzimidazol-2-yl sulphinylmethyl)-4-fluoro-N,N-dimethyl benzenamine
N,N,6-Trimethyl-2-(l-methyl-lH-benzimidazol-2-yl sulphinylmethyl)-benzenamine
2-(l-Methyl-6,7-dihydrodioxino[2,3-f]-lH-benzimidazol-2-yl sulphinylmethyl)-N,N-dimethyl benzenamine,

or

a pharmaceutically acceptable salt of any one thereof.

l0. A compound of formula I, as defined in Claim I above, for use as an intermediate in the synthesis of another chemical compound.

European Patent Office

# EUROPEAN SEARCH REPORT

Application number

EP 87 30 5207

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | GB-A-2 163 747 (NIPPON CHEMIPHAR CO. LTD.)<br>* examples 3-10,12-20,24-26, claims 1,3,5 *<br>--- | 1,6-9 | C 07 D 235/28<br>C 07 D 491/04<br>C 07 D 498/04<br>A 61 K 31/415/<br>C 07 C 43/205<br>C 07 C 79/35<br>C 07 C 95/08<br>C 07 C 91/40 |
| A | EP-A-0 174 717 (FISONS PLC)<br>* examples 1,2j-p, 2s, 2u, 3, 4a-b, 6,18,19; claims 1-9 * & GB - A - 2 161 160 (Cat. D)<br>--- | 1,6-9 | |
| P,X | WO-A-8 701 114 (BYK GULDEN LOMBERG CHEMISCHE FABRIK GMBH)<br>* pages 6-10,12, examples 8,9, claims 1-23,26-30 *<br>--- | 1,5-9 | |
| P,X | EP-A-0 218 336 (NIPPON CHEMIPHAR CO., LTD.)<br>* example 2b; claims *<br>--- | 1,5-9 | TECHNICAL FIELDS SEARCHED (Int. Cl.4) |
| P,A | EP-A-0 213 474 (HOECHST AG)<br>* examples 56,58,69,71,92,94,96,98,99; claims 1-5,8-10,12-16 *<br>--- | 1,6-9 | C 07 D 235/00<br>C 07 D 491/04<br>C 07 D 498/04 |
| P,A | EP-A-0 204 215 (G.D. SEARLE & CO.)<br>* claims 1,9,10,13,15 *<br>----- | 1,6-8 | |

The present search report has been drawn up for all claims

| Place of search | Date of completion of the search | Examiner |
|---|---|---|
| BERLIN | 23-09-1987 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82